# FASCICULE DE BREVET EUROPEEN

(11) **EP 1 047 386 B1**
(45) Date de publication et mention de la délivrance du brevet: **12.04.2006**
(21) Numéro de dépôt: 99901620.7
(22) Date de dépôt: 12.01.1999
(51) Int. Cl.: A61K 8/66, A61Q 5/10

(54) **COMPOSITION DE TEINTURE D'OXYDATION DE FIBRES KERATINIQUES CONTENANT UNE LACCASE ET PROCEDES DE TEINTURE METTANT EN OEUVRE CETTE COMPOSITION**
OXIDATIONSFÄRBEMITTEL FÜR KERATINFASERN, DAS EINE LACCASE ENTHÄLT, UND VERFAHREN ZUR FÄRBUNG MIT DIESEM MITTEL
MIXTURE FOR THE OXIDATION TINTING OF KERATIN FIBRES CONTAINING A LACCASE AND TINTING METHOD USING SAID MIXTURE

(30) Priorité: 13.01.1998 FR 9800251
(43) Date de publication de la demande: 02.11.2000
(73) Titulaire: L'OREAL, 75008 Paris (FR)
(72) Inventeur: LANG, Gérard, F-95390 Saint Prix (FR); COTTERET, Jean, F-78480 Verneuil sur Seine (FR)
(74) Mandataire: Miszputen, Laurent
(86) Numéro de dépôt international: PCT/FR1999/000036
(87) Numéro de publication internationale: WO 1999/036043

(56) Documents cités:
- EP-A- 0 504 005
- EP-A- 0 692 244
- EP-A- 0 958 806
- FR-A- 2 694 018
- FR-A- 2 740 035

## Description

La présente invention a trait à une composition de teinture par oxydation des fibres kératiniques comprenant au moins une enzyme de type laccase, au moins un colorant d'oxydation et au moins un agent de conditionnement des fibres kératiniques particulier et insoluble dans les milieux aqueux, ainsi que ses utilisations pour la teinture des fibres kératiniques en particulier des cheveux humains.

Il est connu de teindre les fibres kératiniques et en particulier les cheveux humains avec des compositions tinctoriales contenant des précurseurs de coloration d'oxydation, en particulier des ortho ou paraphénylènediamines, des ortho ou paraaminophénols, des bases hétérocycliques, appelés généralement bases d'oxydation. Les précurseurs de coloration d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de peroxyde d'hydrogène présente pour inconvénient d'entraîner une dégradation non négligeable des fibres, ainsi qu'une décoloration importante des fibres kératiniques qui n'est pas toujours souhaitable.

La coloration d'oxydation des fibres kératiniques peut également être réalisée à l'aide de systèmes oxydants différents du peroxyde d'hydrogène tels que des systèmes enzymatiques. Ainsi il a déjà été proposé dans le brevet US 3251742, les demandes de brevet FR-A-2 112 549, FR-A-2 694 018, EP-A-0 504 005, WO95/07988. WO95/33836, WO95/33837, WO96/00290, WO97/19998 et WO97/19999 de teindre les fibres kératiniques avec des compositions comprenant au moins un colorant d'oxydation en association avec des enzymes du type laccase ; lesdites compositions étant mises en contact avec l'oxygène de l'air. Ces formulations de teinture, bien qu'étant mises en oeuvre dans des conditions n'entraînant pas une dégradation des fibres kératiniques comparable à celle engendrée par les teintures réalisées en présence de peroxyde d'hydrogène, conduisent à des colorations encore insuffisantes à la fois sur le plan de l'homogénéité de la couleur répartie le long de la fibre (« unisson »), sur le plan de la chromaticité (luminosité) et de la puissance tinctoriale.

On connaît aussi dans la demande EP 958 806 des compositions de coloration pour cheveux comprenant au moins une enzyme laccase et qui se trouvent sous forme de poudre lors de l'application. Ces compositions comprennent en outre un ou plusieurs précurseur de colorants d'oxydation, au moins un tensioactif et /ou au moins un polymère hydrosoluble. La présente invention a pour but de résoudre les problèmes évoqués ci-dessus.

La Demanderesse a découvert de façon surprenante de nouvelles compositions contenant au moins comme système oxydant une enzyme du type laccase et au moins un agent de conditionnement insoluble dans les milieux aqueux particulier que l'on définira plus en détail ci-dessous, pouvant constituer en présence d'au moins un colorant d'oxydation, des formulations de teinture prêtes à l'emploi conduisant à des colorations plus homogènes, plus puissantes et plus chromatiques sans engendrer de dégradation significative, ni de décoloration des fibres kératiniques, peu sélectives et résistant bien aux diverses agressions que peuvent subir les cheveux

Ces découvertes sont à la base de la présente invention.

La présente invention a donc pour premier objet une composition prête à l'emploi, destinée à la coloration par oxydation des fibres kératiniques, en particulier des fibres kératiniques humaines et plus particulièrement les cheveux humains, comprenant dans un support approprié pour les fibres kératiniques :
**(a)** au moins une enzyme du type laccase ;
**(b)** au moins un agent de conditionnement insoluble dans les milieux aqueux choisi dans le groupe constitué par :
   les poly-α-oléfines, les huiles fluorées, les huiles végétales, les cires naturelles, les cires fluorées, les gommes fluorées, les esters d'acides gras choisis parmi les palmitates d'éthyle et d'isopropyle, le palmitate d'éthyl-2-héxyle, le palmitate de 2-octyldécyle, les myristates de butyle, de cétyle, de 2-octyldodécyle, le stéarate d'hexyle, le stéarate de butyle, le malate de dioctyle, le laurate d'hexyle, le laurate de 2-hexyldécyle et l'isononanate d'isononyle, les silicones insolubles, les composés amides comportant au moins une chaîne grasse choisis parmi ceux de formule (1) suivante : 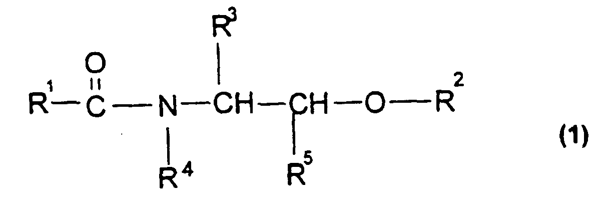
   dans laquelle :
   - R¹ désigne soit un radical hydrocarboné, linéaire ou ramifié, saturé ou insaturé, C₉-C₃₀, ce radical pouvant être substitué par un ou plusieurs groupements hydroxyle éventuellement estérifié par un acide gras saturé ou insaturé en C₁₆-C₃₀ ; soit un radical R"-(NR-CO)ₙ-R' dans lequel n est égal à 0
   ou 1, R désigne hydrogène ou hydroxyéthyle, R' et R" sont des radicaux hydrocarbonés dont la somme des atomes de carbone est comprise entre 9 et 30, R' étant un radical divalent.
   - R² désigne un atome d'hydrogène ou un radical (glycosyle)ₙ, (galactosyle)ₘ ou sulfogalactosyle, dans lesquels n est un entier variant de 1 à 4 et m est un entier variant de 1 à 8 ;
   - R³ désigne un atome d'hydrogène ou un radical hydrocarboné en C₁₆₋C₂₇, saturé ou insaturé, ce radical pouvant être substitué par un ou plusieurs radicaux alkyle en C₁-C₁₄ ; R³ peut également désigner un radical α-hydroxyalkyle en C₁₅-C₂₆, le groupement hydroxyle étant éventuellement estérifié par un α-hydroxyacide en C₁₆-C₃₀;
   - R⁴ désigne un atome d'hydrogène, un radical hydrocarboné en C₁₆-C₂₇. saturé ou insaturé ou un radical -CH₂-CHOH-CH₂-O-R⁶ dans lequel R⁶ désigne un radical hydrocarboné en C₁₀-C₂₆ ;
   - R⁵ désigne un atome d'hydrogène ou un radical hydrocarboné en C₁-C₄ mono ou polyhydroxylé ;
   lesdits agents pouvant être présents sous forme de mélanges ;
**(c)** au moins un colorant d'oxydation.

La ou les laccases utilisées dans la composition tinctoriale prête à l'emploi conforme à l'invention peuvent notamment être choisies parmi les laccases d'origine végétale, d'origine animale, d'origine fongique (levures, moisissures, champignons) ou d'origine bactérienne, les organismes d'origine pouvant être mono ou pluricellulaires. Elles peuvent être obtenues par biotechnologie.

Parmi les laccases d'origine végétale utilisables selon l'invention, on peut citer les laccases produites par des végétaux effectuant la synthèse chlorophyllienne telles qu'indiquées dans la demande FR-A-2 694 018 comme celles que l'on retrouve dans les extraits des Anacardiacées tels que par exemple les extraits de Magnifera indica, Schinus molle ou Pleiogynium timoriense, dans les extraits des Podocarpacées , de Rosmarinus off., de Solanum tuberosum, d'iris sp., de Coffea sp. , de Daucus carrota, de Vinca minor, Persea americana, Catharenthus roseus, Musa sp., Malus pumila, Gingko biloba, Monotropa hypopithys (sucepin), Aesculus sp., Acer pseudoplatanus, Prunus persica, Pistacia palaestina.

Parmi les laccases d'origine fongique éventuellement obtenues par biotechnologie utilisables selon l'invention, on peut citer la ou les laccases issues de Polyporus versicolor, de Rhizoctonia praticola et de Rhus vernicifera comme indiquées dans les demandes FR-A-2 112 549 et EP-A-504005 ; celles décrites dans les demandes de brevet WO95/07988, WO95/33836, WO95/33837, WO96/00290. WO97/19998 et WO97/19999. dont le contenu fait partie intégrante de la présente description comme par exemple celles issues de Scytalidium, de Polyporus pinsitus, de Myceliophtora thermophila, de Rhizoctonia solani, de Pynculana orizae, ou leurs variantes. On peut aussi citer celles issues de Tramates versicolor, de Fomes fomentarius, de Chaetomium thermophile, de Neurospora crassa, de Coriolus versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens et de leurs variantes.

On choisira plus préférentiellement les laccases d'origine fongique éventuellement obtenues par biotechnologie.

L'activité enzymatique des laccases de l'invention ayant la syringaldazine parmi leurs substrats peut être définie à partir de l'oxydation de la syringaldazine en condition aérobie. L'unité lacu correspond à la quantité d'enzyme catalysant la conversion de 1mmole de syringaldazine par minute à pH 5,5 à 30°C. L'unité u correspond à la quantité d'enzyme produisant un delta d'absorbance à 530 nm de 0,001 par minute en utilisant la syringaldazine comme substrat, à 30°C et à pH 6,5.
L'activité enzymatique des laccases de l'invention peut aussi être définie à partir de l'oxydation de la paraphénylènediamine. L'unité ulac correspond à la quantité d'enzyme produisant un delta d'absorbance à 496,5nm de 0,001 par minute en utilisant la paraphénylènediamine comme substrat (64 mM) à 30°C et à pH 5.
Selon l'invention, on préfère déterminer l'activité enzymatique en unités ulac.
Les quantités de laccase utilisées dans les compositions de l'invention varieront en fonction de la nature de la laccase choisie. De façon préférentielle, elles varieront de 0,5 à 2000 lacu, ou de 1000 à 4.10⁷ unités u, ou de 20 à 2.10⁶ unités ulac pour 100g de composition.

Les agents de conditionnement peuvent se présenter sous forme liquide, semi-solide ou solide tels que par exemple des huiles, des cires ou des gommes.

Selon l'invention, parmi les agents de conditionnement, les poly-α-oléfines sont en particulier :
- de type polybutène, hydrogéné ou non, et de préférence polyisobutène, hydrogéné ou non.
   On utilise de préférence les oligomères d'isobutylène de poids moléculaire inférieur à 1000 et leurs mélange avec des polyisobutylènes de poids moléculaire supérieur à 1000 et de préférence compris entre 1000 et 15000.
   A titre d'exemples de poly-α-oléfines utilisables dans le cadre de la présente invention, on peut plus particulièrement mentionner les produits vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non.

De tels produits sont vendus par exemple sous les dénominations ETHYLFLO par la société ETHYL CORP., et d'ARLAMOL PAO par la société ICI.

Les huiles fluorées, les cires fluorées, les gommes fluorées sont par exemple les perfluoropolyéthers décrits notamment dans la demande de brevet EP-A-486135 et les composés fluorohydrocarbonées décrites notamment dans la demande de brevet WO 93/11103. L'enseignement de ces deux demandes est totalement inclus dans la présente demande à titre de référence.

Le terme de composés fluorohydrocarbonées désigne des composés dont la structure chimique comporte un squelette carboné dont certains atomes d'hydrogène ont été substitués par des atomes de fluor.

Les perfluoropolyéthers sont par exemple vendus sous les dénominations commerciales FOMBLIN par la société MONTEFLUOS et KRYTOX par la société DU PONT.

Parmi les composés fluorohydrocarbonés, on peut également citer les esters d'acides gras fluorés tels que le produits vendu sous la dénomination NOFABLE

FO par la société NIPPON OIL.

Selon l'invention, les agents de conditionnement peuvent être choisis parmi les huiles végétales telles que l'huile de jojoba, l'huile d'avocat ou bien les cires naturelles telles que la cire de carnauba ou la cire de pomme.

Selon l'invention, les agents de conditionnement peuvent être choisis parmi les silicones insolubles habituellement utilisées pour améliorer les propriétés cosmétiques des cheveux traités par des formulations capillaires à savoir notamment celles décrites dans les demandes de brevets EP-A- 0181773 et EP-A-0473508.

Il est bien entendu possible de mettre en oeuvre des mélanges de silicones.

Ainsi, selon la présente invention, il est possible d'utiliser toute silicone connue en soi, qu'il s'agisse d'une huile, d'une résine ou bien encore d'une gomme de silicone. Les silicones sont des polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles et en particulier méthyle, les radicaux fluoroalkyles, les radicaux aryles et en particulier phényle, et les radicaux alcényles et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyles ou hydroxyalkyles, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées"). D'une manière générale, les silicones utilisables dans le cadre de la présente invention sont celles qui sont notamment décrites dans "Encyclopedia of Chemical Technology, Kirk-Othmer, Third Edition, 1982, volume 20, pp. 922 et suivantes" et dans "Chemistry and Technology of Silicones, Walter NOLL, Academic Press Inc, San Diego California, 1968". Il est également possible d'utiliser des copolymères blocs linéaires comprenant dans leur chaîne principale des segments polysiloxaniques, comme par exemple des copolymères blocs polysiloxane-polyoxyalkylène ou bien encore polysiloxane-polyuréthane et/ou polyurée. Le poids moléculaire moyen des silicones utilisables selon l'invention peut varier entre 100 et plusieurs millions, de préférence entre 1000 et 1 000 000. Selon la présente invention, on peut bien entendu soit utiliser une seule et même silicone soit, mettre en oeuvre plusieurs silicones différentes.

A titre d'exemples de silicones utilisables dans les compositions selon l'invention, on peut notamment citer les polydialkylsiloxanes, les polyalkylarylsiloxanes, les polydiaryldialkylsiloxanes et d'une manière encore plus générale tous les organopolysiloxanes décrits dans la demande de brevet publiée sous le numéro WO 93/05762 et dont l'enseignement est, à cet égard, totalement inclus dans la présente demande à titre de référence.

Selon un mode de réalisation particulièrement préféré de la présente invention, les silicones utilisées sont choisies parmi les diorganopolysiloxanes (huiles, gommes ou résines), de préférence les polydialkylsiloxanes ou les polyalkylarylsiloxanes, et encore plus préférentiellement les polydiméthylsiloxanes éventuellement modifiés.

Les gommes de silicone sont particulièrement préférées et en particulier celles de polydialkylsiloxanes ou de polyalkylarylsiloxanes. Elles peuvent être utilisées seules ou en mélange dans un solvant choisi par exemple parmi les silicones volatiles, les huiles polydiméthylsiloxane ou polyphénylméthylsiloxane, les isoparaffines, le pentane, le dodécane ou leurs mélanges.

Parmi les composés amides de formule (1), on préfère les céramides et/ou glycocéramides décrites par DOWNING dans Arch. Dermatol, Vol. 123, 1381-1384, 1987, ou celles décrites dans la demande de brevet français FR-2673 79, dont les enseignements sont ici inclus à titre de référence.

Les céramides plus particulièrement préférés selon l'invention sont les composés de formule (1) pour lesquels R¹ désigne un alkyle saturé ou insaturé dérivé d'acides gras en C₁₆-C₂₂ ; R² désigne un atome d'hydrogène ; et R³ désigne un radical linéaire saturé en C₁₅.

De tels composés sont par exemple :
- la N-linoléoyldihydrosphingosine,
- la N-oléoyldihydrosphingosine,
- la N-palmitoyidihydrosphingosine,
- la N-stéaroyldihydrosphingosine,
- la N-béhénoyldihydrosphingosine,
ou les mélanges de ces composés.

On peut également utiliser les composés de formule (1) pour lesquels R¹ désigne un radical alkyle saturé ou insaturé dérivé d'acides gras ; R² désigne un radical galactosyle ou sulfogalactosyle ; et R³ désigne un groupement -CH=CH-(CH₂)₁₂-CH₃.

A titre d'exemple, on peut citer le produit constitué d'un mélange de glycocéramides, vendu sous la dénomination commerciale GLYCOCER par la société WAITAKI INTERNATIONAL BIOSCIENCES.

On peut également utiliser les composés de formule (1) décrits dans les demandes de brevet EP-A-0227994 et WO94/07844.

De tels composés sont par exemple le QUESTAMIDE H (bis-(N-hydroxyéthyl N-cétyl) malonamide) vendu par la société QUEST, le N-(2-hydroxyéthyl)-N-(3-cétyloxy-2-hydroxypropyl)amide d'acide cétylique.

On peut également utiliser le N-docasanoyl N-méthyl-D-glucamine décrit dans la demande de brevet WO92/05764.

Dans la présente invention, on préfère utiliser à titre d'agent de conditionnement insoluble dans les milieux aqueux, les cires naturelles, les huiles végétales, les silicones insolubles et les amides grasses telles que définies ci-avant.

Le ou les agents de conditionnement sont présents dans les compositions conformes à l'invention dans des proportions généralement comprises entre 0,001 et 10% en poids, de préférence de 0,01 à 5 % en poids, et encore plus particulièrement de 0,1 à 2% en poids par rapport au poids total de la composition.

La nature de la ou des colorants d'oxydation utilisées dans la composition tinctoriale prête à l'emploi n'est pas critique. Ils sont choisis parmi les bases d'oxydation et/ou les coupleurs.

Les bases d'oxydation peuvent notamment être choisies parmi les paraphénylènediamines, les bases doubles, les para-aminophénols, les ortho aminophénols et les bases d'oxydation hétérocycliques.

Parmi les paraphénylènediamines utilisables à titre de base d'oxydation dans les composition tinctoriales conformes à l'invention, on peut notamment citer les composés de formule (I) suivante et leurs sels d'addition avec un acide : 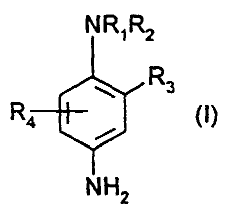 dans laquelle :
- R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁₋C₄), alkyle en C₁-C₄ substitué par un groupement azoté, phényle ou 4'-aminophényle ;
- R₂ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁₋C₄) ou alkyle en C₁-C₄ substitué par un groupement azoté ;
- R₃ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₄ représente un atome d'hydrogène, d'halogène ou un radical alkyle en C₁-C₄.

Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (I) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyt)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (II) suivante, et leurs sels d'addition avec un acide : 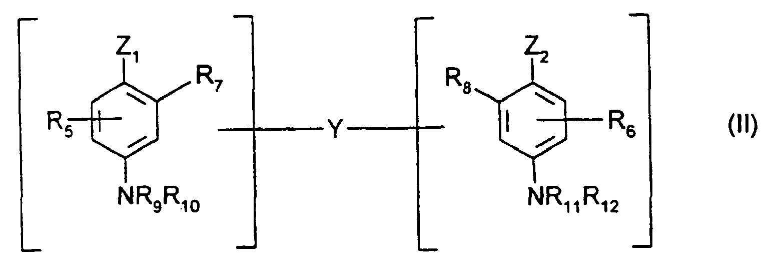 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀ R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (II) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés répondant à la formule (III) suivante, et leurs sels d'addition avec un acide : 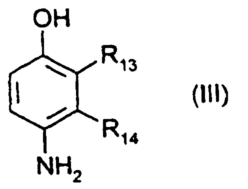 dans laquelle:
- R₁₃ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₄ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄), étant entendu qu'au moins un des radicaux R₁₃ ou R₁₄ représente un atome d'hydrogène.

Parmi les para-aminophénols de formule (III) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, les dérivés pyrazolo-pyrimidiniques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-333 495 ou demandes de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Parmi les dérivés pyrazolo-pyrimidiniques, on peut plus particulièrement citer les pyrazolo-[1,5-a]-pyrimidines de formule (IV) suivante, leurs sels d'addition avec un acide ou avec une base et leurs formes tautomères, lorsqu'il existe un équilibre tautomérique : 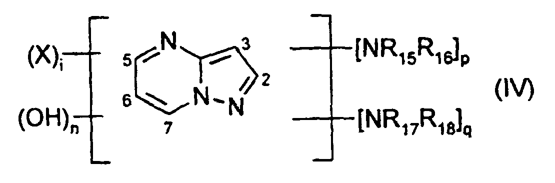 dans laquelle:
- R₁₅, R₁₆, R₁₇ et R₁₈, identiques ou différents désignent, un atome d'hydrogène, un radical alkyle en C₁-C₄, un radical aryle, un radical hydroxyalkyle en C₁-C₄, un radical polyhydroxyalkyle en C₂-C₄, un radical (C₁-C₄)alcoxy alkyle en C₁-C₄, un radical aminoalkyle en C₁-C₄ (l'amine pouvant être protégée par un radical acétyle, uréido ou sulfonyle), un radical (C₁-C₄)alkylamino alkyle en C₁-C₄, un radical di-[(C₁-C₄)alkyl] amino alkyle en C₁-C₄ (les radicaux dialkyles pouvant former un cycle carboné ou un hétérocycle à 5 ou 6 chaînons), un radical hydroxy(C,-C₄)alkyl- ou di-[hydroxy(C₁-C₄) alkyl]-amino alkyle en C₁-C₄ ;
- les radicaux X désignent , identiques ou différents, un atome d'hydrogène, un radical alkyle en C₁-C₄, un radical aryle, un radical hydroxyalkyle en C₁-C₄, un radical polyhydroxyalkyle en C₂-C₄, un radical amino alkyle en C₁-C₄, un radical (C₁-C₄)alkyl amino alkyle en C₁-C₄, un radical di-[(C₁-C₄)alkyl] amino alkyle en C₁-C₄ (les dialkyles pouvant former un cycle carboné ou un hétérocycle à 5 ou 6 chaînons), un radical hydroxy(C₁-C4)alkyl ou di-[hydroxy(C₁-C₄)alkyl]amino alkyle en C₁-C₄, un radical amino, un radical (C₁-C₄)alkyl- ou di-[(C₁-C₄)alkyl]-amino ; un atome d'halogène, un groupe acide carboxylique, un groupe acide sulfonique ;
- i vaut 0, 1, 2 ou 3 ;
- p vaut 0 ou 1 ;
- q vaut 0 ou 1 ;
- n vaut 0 ou 1 ;
sous réserve que :
- la somme p + q est différente de 0 ;
- lorsque p + q est égal à 2, alors n vaut 0 et les groupes NR₁₅R₁₆ et NR₁₇R₁₈ occupent les positions (2,3) ; (5,6) ; (6,7) ; (3,5) ou (3,7) ;
- lorsque p + q est égal à 1 alors n vaut 1 et le groupe NR₁₅R₁₆ (ou NR₁₇R₁₈) et le groupe OH occupent les positions (2,3) ; (5,6) ; (6,7) ; (3,5) ou (3,7) ;

Lorsque les pyrazolo-[1,5-a]-pyrimidines de formule (IV) ci-dessus sont telles qu'elles comportent un groupe hydroxyle sur l'une des positions 2, 5 ou 7 en α d'un atome d'azote, il existe un équilibre tautomérique représenté par exemple par le schéma suivant :

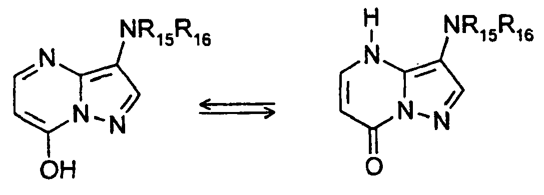

Parmi les pyrazolo-[1,5-a]-pyrimidines de formule (IV) ci-dessus on peut notamment citer :
- la pyrazolo-[1,5-a]-pyrimidine-3,7-diamine ;
- la 2,5-diméthyl pyrazoio-[1,5-a]-pyrimidine-3,7-diamine ;
- la pyrazolo-[1,5-a]-pyrimidine-3,5-diamine ;
- la 2,7-diméthyl pyrazolo-[1,5-a]-pyrimidine-3,5-diamine ;
- le 3-amino pyrazolo-[1,5-a]-pyrimidin-7-ol
- le 3-amino pyrazolo-[1,5-a]-pyrimidin-5-ol
- le 2-(3-amino pyrazolo-[1,5-a]-pyrimidin-7-ylamino)-éthanol
- le 2-(7-amino pyrazolo-[1,5-a]-pyrimidin-3-ylamino)-éthanol
- le 2-[(3-Amino-pyrazolo[1,5-a]pyrimidin-7-yl)-(2-hydroxy-éthyl)-amino]-éthanol
- le 2-[(7-Amino-pyrazolo[1,5-a]pyrimidin-3-yl)-(2-hydroxy-éthyl)-amino]-éthanol
- la 5,6-diméthyl pyrazolo-[1,5-a]-pyrimidine-3,7-diamine ;
- la 2,6-diméthyl pyrazolo-[1,5-a]-pyrimidine-3,7-diamine ;
- la 2, 5, N 7, N 7-tetraméthyl pyrazolo-[1,5-a]-pyrimidine-3,7-diamine ;
et leurs sels d'addition et leurs formes tautomères, lorsqu'il existe un équilibre tautomérique.

Les pyrazolo-[1,5-a]-pyrimidines de formule (IV) ci-dessus peuvent être préparées par cyclisation à partir d'un aminopyrazole selon les synthèses décrites dans les références suivantes :
- EP 628559 BEIERSDORF-LILLY
- R. Vishdu, H. Navedul, Indian J. Chem., 34b (6), 514, 1995.
- N.S. Ibrahim, K.U. Sadek, F.A. Abdel-Al, Arch. Pharm., 320, 240, 1987.
- R.H. Springer, M.B. Scholten, D.E. O'Brien, T. Novinson, J.P. Miller, R.K. Robins, J. Med. Chem., 25, 235, 1982.
- T. Novinson, R.K. Robins, T.R. Matthews, J. Med. Chem., 20, 296, 1977.
- US 3907799 ICN PHARMACEUTICALS

Les pyrazolo-[1,5-a]-pyrimidines de formule (IV) ci-dessus peuvent également être préparées par cyclisation à partir d'hydrazine selon les synthèses décrites dans les références suivantes :
- A. McKillop et R.J. Kobilecki, Heterocycles, 6(9), 1355, 1977.
- E. Alcade, J. De Mendoza, J.M. Marcia-Marquina, C. Almera, J. Elguero, J. Heterocyclic Chem., 11(3). 423, 1974.
- K. Saito, I. Hori, M. Higarashi, H. Midorikawa, Bull. Chem. Soc. Japan, 47(2), 476, 1974.

La ou les bases d'oxydation représentent de préférence de 0,0005 à 12 % en poids environ du poids total de la composition tinctoriale conforme à l'invention, et encore plus préférentiellement de 0,005 à 6 % en poids environ de ce poids.

Le ou les coupleurs pouvant être utilisés dans la composition tinctoriale prête à l'emploi conforme à l'invention sont ceux classiquement utilisés dans les compositions de teinture d'oxydation, c'est-à-dire des méta-phénylènediamines, des méta-aminophénols, des métadiphénols, des coupleurs hétérocycliques, et leurs sels d'addition avec un acide.

Ces coupleurs peuvent notamment être choisis parmi le 2-méthyl-5-amino-phénol, le 5-N-(β-hydroxyéthyl)-amino-2-méthyl-phénol, le 3-amino-phénol, le 1,3-dihydroxybenzène, le 1,3-dihydroxy-2-méthyl-benzène, le 4-chloro-9,3-dihydroxy-benzène, le 2,4-diamino-1-(β-hydroxyéthyloxy)-benzène, le 2-amino-4-(β-hydroxyéthylamino)-1-méthoxy-benzène, le 1,3-diamino-benzène, le 1,3-bis-(2,4-diaminophénoxy)-propane, le sésamol, l'α-naphtol, le 6-hydroxy-indole, le 4-hydroxy-indole, le 4-hydroxy-N-méthyl-indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, le 2,6-diméthylpyrazolo-[1,5-b]-1,2,4-triazote, le 2,6-diméthyl-[3,2-c]-1,2,4-triazole, le 6-méthylpyrazolo-[1,5-a]-benzimidazole, et leurs sels d'addition avec un acide.

Ces coupleurs représentent de préférence de 0,0001 à 10 % en poids environ du poids total de la composition tinctoriale prête à l'emploi et encore plus préférentiellement de 0,005 à 5 % en poids environ de ce poids.

D'une manière générale, les sels d'addition avec un acide des colorants d'oxydation utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

La composition tinctoriale de l'invention peut encore contenir, en plus des colorants d'oxydation définis ci-dessus, des colorants directs pour enrichir les nuances en reflets. Ces colorants directs peuvent notamment alors être choisis parmi les colorants nitrés, azoïques ou anthraquinoniques.

La composition tinctoriale prête à l'emploi conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères, des agents épaississants, des agents antioxydants, des enzymes différentes des laccases utilisées conformément à l'invention telles que par exemple des peroxydases ou des oxydo-réductases à 2 électrons, des agents de pénétration, des agents séquestrants, des parfums, des tampons, des agents dispersants, des agents filmogènes, des agents filtrants, des vitamines, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains. Dans ce cas, le ou les colorants d'oxydation et la ou les laccases sont présents au sein de la même composition prête à l'emploi, et par conséquent ladite composition doit être exempte d'oxygène gazeux, de manière à éviter toute oxydation prématurée du ou des colorants d'oxydation.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une forme de réalisation particulière de l'invention, le procédé comporte une étape préliminaire consistant à stocker sous forme séparée, d'une part, une composition (A) comprenant, dans un milieu approprié pour la teinture, au moins un colorant d'oxydation tel que défini précédemment et, d'autre part, une composition (B) renfermant, dans un milieu approprié pour la teinture, au moins une enzyme de type laccase et au moins un agent de conditionnement insoluble dans les milieux aqueux tel que défini précédemment,
puis à procéder à leur mélange au moment de l'emploi avant d'appliquer ce mélange sur les fibres kératiniques.

Selon une forme de réalisation particulière de l'invention, l'agent de conditionnement insoluble peut être incorporé dans la composition (A).

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Le milieu approprié pour les fibres kératiniques (ou support) des compositions tinctoriales prêtes à l'emploi des fibres kératiniques conformes à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ainsi que les alcools aromatiques comme l'alcool benzylique, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH des compositions tinctoriales prêtes à l'emploi des fibres kératiniques conformes à l'invention est choisi de telle manière que l'activité enzymatique de la laccase ne soit pas altérée. Il varie plus préférentiellement de 6 à 9 environ.

Des exemples concrets illustrant l'invention vont maintenant être donnés.

Dans ce qui suit ou ce qui précède, sauf mention contraire, les pourcentages sont exprimés en poids. Les exemples suivants illustrent l'invention sans présenter un caractère limitatif.

### EXEMPLE: Composition de teinture

On a préparé la composition tinctoriale prête à l'emploi suivante (teneurs en grammes) :
- Laccase issue de Rhus vemicifera à 180 unités/mg commercialisée par la société SIGMA 1,8g
- Alkyl (C₈-C₁₀) polyglucoside en solution aqueuse à 60 % de matière active (M.A.) vendu sous la dénomination ORAMIX CG110 par la société SEPPIC 16,5 g
- Paraphénylènediamine 0,254 g
- Dichlorhydrate de 2,4-diarninophénoxyéthanol 0,260 g
- Diméthicone vendue sous le nom MIRASIL DM 500000 par la société RHONE POULENC 0,5 g
- Ethanol 20,0 g
- Agent de pH qs pH 6,5
- Eau déminéralisée qsp 100 g

Cette composition tinctoriale prête à l'emploi a été appliquée sur des mèches de cheveux gris naturels à 90 % de blancs pendant 40 minutes à 30°C. Les cheveux ont ensuite été rincés, lavés avec un shampooing standard, puis séchés.

On obtient des mèches de cheveux teintes en gris bleuté.

Dans cet exemple, 1,8 g de laccase issue de Rhus vemicifera à 180 unités/mg peut être remplacé par 1g de laccase issue de Pyricularia Orizae à 100 unités/mg vendue par la société I.C.N.

## Revendications (Revendications pour l'(les) Etat(s) contractant(s) suivant(s): AT, DK, GR, IE, MC, PT, SE)

1. Composition prête à l'emploi, de pH variant de 4 à 11, pour la teinture par oxydation des fibres kératiniques, en particulier des fibres kératiniques humaines et plus particulièrement les cheveux humains, comprenant dans un support approprié pour la teinture des fibres kératiniques :
**(a)** au moins une enzyme du type laccase ;
**(b)** au moins un agent de conditionnement insoluble dans les milieux aqueux choisi dans le groupe constitué par :
les poly-α-oléfines, les huiles fluorées, les huiles végétales, les cires naturelles, les cires fluorées, les gommes fluorées, les esters d'acides gras choisis parmi les palmitates d'éthyle et d'isopropyle, le palmitate d'éthyl-2-héxyle, le palmitate de 2-octyldécyle, les myristates de butyle, de cétyle, de 2-octyldodécyle, le stéarate d'hexyle, le stéarate de butyle, le malate de dioctyle, le laurate d'hexyle, le laurate de 2-hexyldécyle et l'isononanate d'isononyle, les silicones insolubles, les composés amides comportant au moins une chaîne grasse choisis parmi ceux de formule (1) suivante : 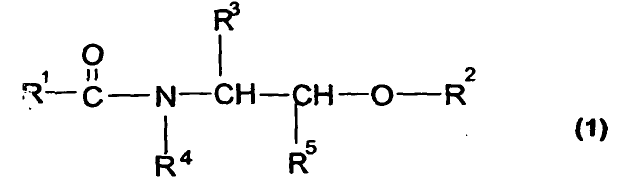
dans laquelle :
- R¹ désigne soit un radical hydrocarboné, linéaire ou ramifié, saturé ou insaturé, C₉-C₃₀, ce radical pouvant être substitué par un ou plusieurs groupements hydroxyle éventuellement estérifié par un acide gras saturé ou insaturé en C₁₆-C₃₀ ; soit un radical R"-(NR-CO)ₙ-R' dans lequel n est égal à 0
ou 1, R désigne hydrogène ou hydroxyéthyle, R' et R" sont des radicaux hydrocarbonés dont la somme des atomes de carbone est comprise entre 9 et 30, R' étant un radical divalent.
- R² désigne un atome d'hydrogène ou un radical (glycosyle)ₙ, (galactosyle)ₘ ou sulfogalactosyle, dans lesquels n est un entier variant de 1 à 4 et m est un entier variant de 1 à 8 ;
- R³ désigne un atome d'hydrogène ou un radical hydrocarboné en C₁₆₋C₂₇, saturé ou insaturé, ce radical pouvant être substitué par un ou plusieurs radicaux alkyle en C₁-C₁₄ ; R³ peut également désigner un radical α-hydroxyalkyle en C₁₅-C₂₆, le groupement hydroxyle étant éventuellement estérifié par un α-hydroxyacide en C₁₆-C₃₀ ;
- R⁴ désigne un atome d'hydrogène, un radical hydrocarboné en C₁₆-C₂₇, saturé ou insaturé ou un radical -CH₂-CHOH-CH₂-O-R⁶ dans lequel R⁶ désigne un radical hydrocarboné en C₁₀-C₂₆ ;
- R⁵ désigne un atome d'hydrogène ou un radical hydrocarboné en C₁-C₄ mono ou polyhydroxylé ;
lesdits agents pouvant être présents sous forme de mélanges ;
**(c)** au moins un colorant d'oxydation.

2. Composition selon la revendication 1, **caractérisée par le fait que** la ou les laccases sont choisies parmi les laccases d'origine végétale, d'origine animale, d'origine fongique, d'origine bactérienne, ou obtenues par biotechnologie.

3. Composition selon l'une quelconque des revendications 1 à 2, où les laccases sont choisies parmi celles produites par des végétaux effectuant la synthèse chlorophyllienne.

4. Composition selon la revendication 3, où les laccases sont choisies parmi celles extraites des Anacardiacées ou des Podocarpacées , de Rosmarinus off., de Solanum tuberosum, d'Iris sp., de Coffea sp., de Daucus carrota, de Vinca minor, Persea americana, Catharenthus roseus, Musa sp., Malus pumila, Gingko biloba, Monotropa hypopithys (sucepin), Aesculus sp., Acer pseudoplatanus, Prunus persica, Pistacia palaestina.

5. Composition selon la revendication 2, où les laccases sont choisies parmi celles issues de Pyricularia orizae, de Polyporus versicolor, de Rhizoctonia praticola, de Rhus vemicifera, de Scytalidium, de Polyporus pinsitus, de Myceliophtora thermophila, de Rhizoctonia solani, de Tramates versicolor, de Fomes fomentarius, de Chaetomium thermophile, de Neurospora crassa, de Coriolus versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, ainsi que leurs variantes.

6. Composition selon l'une quelconque des revendications 1 à 5, **caractérisée par le fait que** la ou les laccases sont présentes dans des quantités allant de 0,5 à 2000 lacu, ou de 1000 à 4.10⁷ unités u, ou de 20 à 2.10⁶ unités ulac, pour 100g de composition.

7. Composition selon la revendication 1, selon laquelle les agents de conditionnement se présentent sous forme liquide, semi-solide ou solide et préférence sous forme d'huile, de cire ou de gomme.

8. Composition selon les revendications 1 ou 7, selon lesquelles les agents de conditionnement sont choisis parmi les huiles végétales, les cires naturelles, les silicones insolubles et les composés amides de formule (1).

9. Composition selon les revendications 1 ou 7, selon lesquelles les poly-α-oléfines sont choisies parmi celles :
- de type polybutène, hydrogéné ou non, et de préférence polyisobutène, hydrogéné ou non ;
- de type polydécène, hydrogéné ou non.

10. Composition selon les revendications 1 ou 7, selon lesquelles les silicones sont choisies parmi les polydialkylsiloxanes, les polyalkylarylsiloxanes, les polydiaryldialkylsiloxanes éventuellement modifiés, de préférence les polydialkylsiloxanes ou les polyalkylarylsiloxanes, et encore plus préférentiellement les polydiméthylsiloxanes éventuellement modifiés.

11. Composition selon la revendication 10, où les silicones sont choisies parmi les gommes de polydialkylsiloxanes , les gommes de polyalkylarylsiloxanes et sont utilisées seules ou en mélanges dans un solvant.

12. Composition selon l'une quelconque des revendications 1, 7 ou 8, **caractérisée par le fait que** dans la formule (1) des composés amides, R¹ désigne un alkyle saturé ou insaturé dérivé d'acides gras en C₁₆-C₂₂ ; R² désigne un atome d'hydrogène ; et R³ désigne un radical linéaire saturé en C₁₅.

13. Composition selon la revendication 1 ou 12, où les composés amides sont choisis parmi :
- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
ou les mélanges de ces composés.

14. Composition selon l'une quelconque des revendications 1, 7 ou 8, **caractérisée par le fait que** dans la formule (1) des composés amides, R¹ désigne un radical alkyle saturé ou insaturé dérivé d'acides gras ; R² désigne un radical galactosyle ou sulfogalactosyle ; et R³ désigne un groupement -CH=CH-(CH₂)₁₂-CH₃.

15. Composition selon l'une quelconque des revendications 1 et 7 à 14, où les agents de conditionnement sont présents dans des proportions allant de 0,001 à 10% en poids, de préférence de 0,01 à 5 % en poids, et encore plus particulièrement de 0,1 à 2% en poids par rapport au poids total de la composition.

16. Composition selon la revendication 1, **caractérisée par le fait que** les colorants d'oxydation sont des bases d'oxydation choisies parmi les ortho- ou para- phénylènediamines, les bis-phénylalkylènediamines, les ortho- ou para- aminophénols, et les bases hétérocycliques, ainsi que les sels d'addition de ces composés avec un acide.

17. Composition selon la revendication 16, **caractérisée par le fait que** les bases d'oxydation sont présentes dans des concentrations allant de 0,0005 à 12% en poids par rapport au poids total de la composition.

18. Composition selon la revendication 1, **caractérisée par le fait que** les colorants d'oxydation sont des coupleurs choisis parmi les métaphénylènediamines, les métaaminophénols, les métadiphénols, les coupleurs hétérocycliques, et les sels d'addition de ces composés avec un acide.

19. Composition selon la revendication 18, **caractérisée par le fait que** les coupleurs sont présents dans des concentrations allant de 0,0001 à 10% en poids par rapport au poids total de la composition.

20. Composition selon l'une quelconque des revendications 1 et 16 à 19, **caractérisée par le fait que** les sels d'addition avec un acide des colorants sont choisis parmi les chlorhydrates, les bromhydrates, les sulfates, les tartrates, les lactates et les acétates.

21. Composition selon l'une quelconque des revendications précédentes, **caractérisée par le fait qu'**elle contient en outre des colorants directs.

22. Composition selon l'une quelconque des revendications précédentes, **caractérisée par le fait que** le milieu approprié pour les fibres kératiniques (ou support) est constitué par de l'eau ou par un mélange d'eau et d'au moins un solvant organique.

23. Composition selon la revendication 22, **caractérisée par le fait que** les solvants organiques peuvent être présents dans des proportions de préférence allant 1 à 40 % en poids par rapport au poids total de la composition, et encore plus préférentiellement allant de 5 à 30 % en poids.

24. Composition selon l'une quelconque des revendications précédentes, **caractérisée par le fait que** le pH varie de 6 à 9 environ.

25. Composition selon l'une quelconque des revendications précédentes, **caractérisée par le fait qu'**elle contient en plus au moins un adjuvant cosmétique utilisé classiquement dans les compositions pour la teinture, choisi dans le groupe constitué par des agents tensio-actifs, des polymères, des agents épaississants, des agents antioxydants, des enzymes différentes des laccases, des agents de pénétration, des agents séquestrants, des parfums, des tampons, des agents dispersants, des agents filmogènes, des agents filtrants, des vitamines, des agents conservateurs, des agents opacifiants.

26. Procédé de teinture des fibres kératiniques et en particulier des fibres kératiniques humaines telles que les cheveux, **caractérisé par le fait qu'**on applique sur lesdites fibres au moins une composition tinctoriale prête à l'emploi telle que définie dans l'une quelconque des revendications 1 à 25, pendant un temps suffisant pour développer la coloration désirée.

27. Procédé selon la revendication 26, **caractérisé par le fait qu'**il comporte une étape préliminaire consistant à stocker sous forme séparée, d'une part, une composition (A) comprenant, dans un milieu approprié pour la teinture, au moins un colorant d'oxydation tel que défini dans l'une quelconque des revendications 1 et 16 à 20 et d'autre part, une composition (B) renfermant, dans un milieu approprié pour les fibres kératiniques, au moins une enzyme du type laccase telle que définie dans l'une quelconque des revendications 1 à 6, puis à procéder à leur mélange au moment de l'emploi avant d'appliquer ce mélange sur les fibres kératiniques ; la composition (A) ou la composition (B) contenant l'agent de conditionnement tel que défini dans les revendications 1 et 7 à 14.

28. Dispositif à plusieurs compartiments ou "kit" de teinture, **caractérisé par le fait qu'**il comporte un premier compartiment renfermant la composition (A) telle que définie dans la revendication 27 et un second compartiment renfermant la composition (B) telle que définie dans la revendication 27 ; la composition (A) ou la composition (B) contenant l'agent de conditionnement tel que défini dans les revendications 1 et 7 à 14.

## Revendications (Revendications pour l'(les) Etat(s) contractant(s) suivant(s): BE, CH, LI, DE, ES, FR, GB, IT, NL)

1. Composition prête à l'emploi de pH variant de 4 à 11, pour la teinture par oxydation des fibres kératiniques, en particulier des fibres kératiniques humaines et plus particulièrement les cheveux humains, comprenant dans un support approprié pour la teinture des fibres kératiniques :
**(a)** au moins une enzyme du type laccase ;
**(b)** au moins un agent de conditionnement insoluble dans les milieux aqueux choisi dans le groupe constitué par :
les poly-α-oléfines, les huiles fluorées, les huiles végétales, les cires naturelles, les cires fluorées, les gommes fluorées, les esters d'acides gras choisis parmi les palmitates d'éthyle et d'isopropyle, le palmitate d'éthyl-2-héxyle, le palmitate de 2-octyldécyle, les myristates de butyle, de cétyle, de 2-octyldodécyle, le stéarate d'hexyle, le stéarate de butyle, le malate de dioctyle, le laurate d'hexyle, le laurate de 2-hexyldécyle et l'isononanate d'isononyle, les silicones insolubles, les composés amides comportant au moins une chaîne grasse choisis parmi ceux de formule (1) suivante : 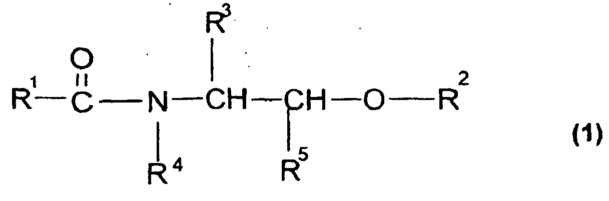
dans laquelle :
- R¹ désigne soit un radical hydrocarboné, linéaire ou ramifié, saturé ou insaturé, C₉-C₃₀, ce radical pouvant être substitué par un ou plusieurs groupements hydroxyle éventuellement estérifié par un acide gras saturé ou insaturé en C₁₆-C₃₀ ; soit un radical R"-(NR-CO)ₙ-R' dans lequel n est égal à 0
ou 1, R désigne hydrogène ou hydroxyéthyle, R' et R" sont des radicaux hydrocarbonés dont la somme des atomes de carbone est comprise entre 9 et 30, R' étant un radical divalent.
- R² désigne un atome d'hydrogène ou un radical (glycosyle)ₙ, (galactosyle)ₘ ou sulfogalactosyle, dans lesquels n est un entier variant de 1 à 4 et m est un entier variant de 1 à 8 ;
- R³ désigne un atome d'hydrogène ou un radical hydrocarboné en C₁₆₋C₂₇, saturé ou insaturé, ce radical pouvant être substitué par un ou plusieurs radicaux alkyle en C₁-C₁₄ ; R³ peut également désigner un radical α-hydroxyalkyle en C₁₅-C₂₆, le groupement hydroxyle étant éventuellement estérifié par un α-hydroxyacide en C₁₆-C₃₀ ;
- R⁴ désigne un atome d'hydrogène, un radical hydrocarboné en C₁₆-C₂₇, saturé ou insaturé ou un radical -CH₂-CHOH-CH₂-O-R⁶ dans lequel R⁶ désigne un radical hydrocarboné en C₁₀-C₂₆;
- R⁵ désigne un atome d'hydrogène ou un radical hydrocarboné en C₁-C₄ mono ou polyhydroxylé ;
lesdits agents pouvant être présents sous forme de mélanges ;
**(c)** au moins un colorant d'oxydation ;
A l'exclusion de la composition suivante :
| | Quantité (% poids) |
|---|---|
| Toluène-2,5-diamine | 2.0 |
| Para-aminophénol | 1.0 |
| Méta-aminophénol | 0.03 |
| Laccase | 0.3 |
| Méthyl-polysiloxane (poids - poids moléculaire moyen: 100,000) | 4.5 |
| Méthyl-polysitoxane (30 cs) | 2.5 |
| N-cocoyl-L-glutamate triéthanolamine | 0.5 |
| Yukaformer 301 | 0.9 |
| Ethanol | 5.0 |
| Eau pure | Qsp 100 |
| Total | 100.0 |

2. Composition prête à l'emploi de pH variant de 4 à 11, pour la teinture par oxydation des fibres kératiniques, en particulier des fibres kératiniques humaines et plus particulièrement les cheveux humains, comprenant dans un support approprié pour la teinture des fibres kératiniques :
**(a)** au moins une enzyme du type laccase ;
**(b)** au moins un agent de conditionnement insoluble dans les milieux aqueux choisi dans le groupe constitué par :
les poly-α-oléfines ; les huiles fluorées ; les huiles végétales ; les cires naturelles ; les cires fluorées ; les gommes fluorées ; les esters d'acides gras choisis parmi les palmitates d'éthyle et d'isopropyle, le palmitate d'éthyl-2-héxyle, le palmitate de 2-octyldécyle, les myristates de butyle, de cétyle, de 2-octyldodécyle, le stéarate d'hexyle, le stéarate de butyle, le malate de dioctyle, le laurate d'hexyle, le laurate de 2-hexyldécyle et l'isononanate d'isononyle ; les silicones insolubles choisies parmi les polydialkylsiloxanes modifiés, les polyalkylarylsiloxanes, les polydiaryldialkylsiloxanes éventuellement modifiés ; les composés amides comportant au moins une chaîne grasse choisis parmi ceux de formule (1) suivante : 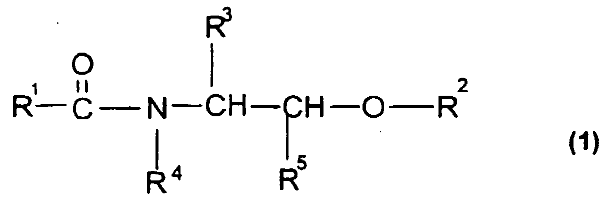
dans laquelle :
- R¹ désigne soit un radical hydrocarboné, linéaire ou ramifié, saturé ou insaturé, C₉-C₃₀, ce radical pouvant être substitué par un ou plusieurs groupements hydroxyle éventuellement estérifié par un acide gras saturé ou insaturé en C₁₆-C₃₀ ; soit un radical R"-(NR-CO)ₙ-R' dans lequel n est égal à 0
ou 1, R désigne hydrogène ou hydroxyéthyle, R' et R" sont des radicaux hydrocarbonés dont la somme des atomes de carbone est comprise entre 9 et 30, R' étant un radical divalent.
- R² désigne un atome d'hydrogène ou un radical (glycosyle)ₙ, (galactosyle)ₘ ou sulfogalactosyle, dans lesquels n est un entier variant de 1 à 4 et m est un entier variant de 1 à 8 ;
- R³ désigne un atome d'hydrogène ou un radical hydrocarboné en C₁₆₋C₂₇, saturé ou insaturé, ce radical pouvant être substitué par un ou plusieurs radicaux alkyle en C₁-C₁₄ ; R³ peut également désigner un radical α-hydroxyalkyle en C₁₅-C₂₆, le groupement hydroxyle étant éventuellement estérifié par un α-hydroxyacide en C₁₆-C₃₀ ;
- R⁴ désigne un atome d'hydrogène, un radical hydrocarboné en C₁₆-C₂₇, saturé ou insaturé ou un radical -CH₂-CHOH-CH₂-O-R⁶ dans lequel R⁶ désigne un radical hydrocarboné en C₁₀-C₂₆ ;
- R⁵ désigne un atome d'hydrogène ou un radical hydrocarboné en C₁-C₄ mono ou polyhydroxylé ;
lesdits agents pouvant être présents sous forme de mélanges ;
**(c)** au moins un colorant d'oxydation

3. Composition selon l'une quelconque des revendications 1 à 2, **caractérisée par le fait que** la ou les laccases sont choisies parmi les laccases d'origine végétale, d'origine animale, d'origine fongique, d'origine bactérienne, ou obtenues par biotechnologie.

4. Composition selon l'une quelconque des revendications 1 à 3, où les laccases sont choisies parmi celles produites par des végétaux effectuant la synthèse chlorophyllienne.

5. Composition selon la revendication 4, où les laccases sont choisies parmi celles extraites des Anacardiacées ou des Podocarpacées , de Rosmarinus off., de Solanum tuberosum, d'Iris sp., de Coffea sp., de Daucus carrota, de Vinca minor, Persea americana, Catharenthus roseus, Musa sp., Malus pumila, Gingko biloba, Monotropa hypopithys (sucepin), Aesculus sp., Acer pseudoplatanus, Prunus persica, Pistacia palaestina.

6. Composition selon la revendication 3, où les laccases sont choisies parmi celles issues de Pyricularia orizae, de Polyporus versicolor, de Rhizoctonia praticola, de Rhus vernicifera, de Scytalidium, de Polyporus pinsitus, de Myceliophtora thermophila, de Rhizoctonia solani, de Tramates versicolor, de Fomes fomentarius, de Chaetomium thermophile, de Neurospora crassa, de Coriolus versicol, de Botrytis cinerea, de Rigidoporus lignosus, de Phellinus noxius, de Pleurotus ostreatus; d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, ainsi que leurs variantes.

7. Composition selon l'une quelconque des revendications 1 à 6, **caractérisée par le fait que** la ou les laccases sont présentes dans des quantités allant de 0,5 à 2000 lacu, ou de 1000 à 4.10⁷ unités u, ou de 20 à 2.10⁶ unités ulac, pour 100g de composition.

8. Composition selon la revendication 1 ou 2, selon laquelle les agents de conditionnement se présentent sous forme liquide, semi-solide ou solide et préférence sous forme d'huile, de cire ou de gomme.

9. Composition selon les revendications 1, 2 ou 8, selon lesquelles les agents de conditionnement sont choisis parmi les huiles végétales, les cires naturelles, les silicones insolubles et les composés amides de formule (1).

10. Composition selon les revendications 1, 2 ou 8, selon lesquelles les poly-α-oléfines sont choisies parmi celles :
- de type polybutène, hydrogéné ou non, et de préférence polyisobutène, hydrogéné ou non ;
- de type polydécène, hydrogéné ou non.

11. Composition selon les revendications 1 ou 8, selon lesquelles les silicones sont choisies parmi les polydialkylsiloxanes, les polyalkylarylsiloxanes, les polydiaryldialkylsiloxanes éventuellement modifiés, de préférence les polydialkylsiloxanes ou les polyalkylarylsiloxanes, et encore plus préférentiellement les polydiméthylsiloxanes éventuellement modifiés.

12. Composition selon la revendication 11, où les silicones sont choisies parmi les gommes de polydialkylsiloxanes, les gommes de polyalkylarylsiloxanes et sont utilisées seules ou en mélanges dans un solvant.

13. Composition selon les revendications 2 ou 8, selon lesquelles les silicones sont choisies parmi les polydialkylsiloxanes modifiés, les polyalkylarylsiloxanes, éventuellement modifiés.

14. Composition selon la revendication 13, où les silicones sont choisies parmi les gommes de polyalkylarylsiloxanes et sont utilisées seules ou en mélanges dans un solvant.

15. Composition selon l'une quelconque des revendications 1, 2, 8 ou 9, **caractérisée par le fait que** dans la formule (1) des composés amides, R¹ désigne un alkyle saturé ou insaturé dérivé d'acides gras en C₁₆-C₂₂ ; R² désigne un atome d'hydrogène ; et R³ désigne un radical linéaire saturé en C₁₅.

16. Composition selon la revendication 1 ou 15, où les composés amides sont choisis parmi :
- la N-linoléoyldihydrosphingosine,
- la N-oléoyldihydrosphingosine,
- la N-palmitoyldihydrosphingosine,
- la N-stéaroyldihydrosphingosine,
- la N-béhénoyldihydrosphingosine,
ou les mélanges de ces composés.

17. Composition selon l'une quelconque des revendications 1, 2, 8 ou 9, **caractérisée par le fait que** dans la formule (1) des composés amides, R¹ désigne un radical alkyle saturé ou insaturé dérivé d'acides gras ; R² désigne un radical galactosyle ou sulfogalactosyle ; et R³ désigne un groupement -CH=CH-(CH₂)₁₂-CH₃.

18. Composition selon l'une quelconque des revendications 1, 2 et 8 à 17, où les agents de conditionnement sont présents dans des proportions allant de 0,001 à 10% en poids, de préférence de 0,01 à 5 % en poids, et encore plus particulièrement de 0,1 à 2% en poids par rapport au poids total de la composition.

19. Composition selon l'une quelconque des revendications 1 ou 2, **caractérisée par le fait que** les colorants d'oxydation sont des bases d'oxydation choisies parmi les ortho- ou para- phénylènediamines, les bis-phénylalkylènediamines, les ortho- ou para- aminophénols, et les bases hétérocycliques, ainsi que les sels d'addition de ces composés avec un acide.

20. Composition selon la revendication 19, **caractérisée par le fait que** les bases d'oxydation sont présentes dans des concentrations allant de 0,0005 à 12% en poids par rapport au poids total de la composition.

21. Composition selon l'une quelconque des revendications 1 ou 2, **caractérisée par le fait que** les colorants d'oxydation sont des coupleurs choisis parmi les métaphénylènediamines, les métaaminophénols, les métadiphénols, les coupleurs hétérocycliques, et les sels d'addition de ces composés avec un acide.

22. Composition selon la revendication 21, **caractérisée par le fait que** les coupleurs sont présents dans des concentrations allant de 0,0001 à 10% en poids par rapport au poids total de la composition.

23. Composition selon l'une quelconque des revendications 1 et 19 à 22, **caractérisée par le fait que** les sels d'addition avec un acide des colorants sont choisis parmi les chlorhydrates, les bromhydrates, les sulfates, les tartrates, les lactates et les acétates.

24. Composition selon l'une quelconque des revendications précédentes, **caractérisée par le fait qu'**elle contient en outre des colorants directs.

25. Composition selon l'une quelconque des revendications précédentes, **caractérisée par le fait que** le milieu approprié pour les fibres kératiniques (ou support) est constitué par de l'eau ou par un mélange d'eau et d'au moins un solvant organique.

26. Composition selon la revendication 25, **caractérisée par le fait que** les solvants organiques peuvent être présents dans des proportions de préférence allant 1 à 40 % en poids par rapport au poids total de la composition, et encore plus préférentiellement allant de 5 à 30 % en poids.

27. Composition selon l'une quelconque des revendications précédentes, **caractérisée par le fait que** le pH varie de 6 à 9 environ.

28. Composition selon l'une quelconque des revendications précédentes, **caractérisée par le fait qu'**elle contient en plus au moins un adjuvant cosmétique utilisé classiquement dans les compositions pour la teinture, choisi dans le groupe constitué par des agents tensio-actifs, des polymères, des agents épaississants, des agents antioxydants, des enzymes différentes des laccases, des agents de pénétration, des agents séquestrants, des parfums, des tampons, des agents dispersants, des agents filmogènes, des agents filtrants, des vitamines, des agents conservateurs, des agents opacifiants.

29. Procédé de teinture des fibres kératiniques et en particulier des fibres kératiniques humaines telles que les cheveux, **caractérisé par le fait qu'**on applique sur lesdites fibres au moins une composition tinctoriale prête à l'emploi telle que définie dans l'une quelconque des revendications 1 à 28, pendant un temps suffisant pour développer la coloration désirée.

30. Procédé selon la revendication 29, **caractérisé par le fait qu'**il comporte une étape préliminaire consistant à stocker sous forme séparée, d'une part, une composition (A) comprenant, dans un milieu approprié pour la teinture, au moins un colorant d'oxydation tel que défini dans l'une quelconque des revendications 1, 2 et 19 à 23 et d'autre part, une composition (B) renfermant, dans un milieu approprié pour les fibres kératiniques, au moins une enzyme du type laccase telle que définie dans l'une quelconque des revendications 1 à 7, puis à procéder à leur mélange au moment de l'emploi avant d'appliquer ce mélange sur les fibres kératiniques ; la composition (A) ou la composition (B) contenant l'agent de conditionnement tel que défini dans l'une quelconque des revendications 1, 2 et 8 à 18.

31. Dispositif à plusieurs compartiments ou "kit" de teinture, **caractérisé par le fait qu'**il comporte un premier compartiment renfermant la composition (A) telle que définie dans la revendication 30 et un second compartiment renfermant la composition (B) telle que définie dans la revendication 30 ; la composition (A) ou la composition (B) contenant l'agent de conditionnement tel que défini dans les revendications 1, et 8 à 18.

## Claims (Claims for the following Contracting State(s): AT, DK, GR, IE, MC, PT, SE)

1. Ready-to-use composition with a pH varying from 4 to 11 for the oxidation dyeing of keratinous fibres, in particular human keratinous fibres and more particularly human hair, comprising, in a carrier appropriate for dyeing keratinous fibres:
(a) at least one enzyme of the laccase type;
(b) at least one conditioning agent insoluble in aqueous media, chosen from the group consisting of:
poly-α-olefins, fluorinated oils, vegetable oils, natural waxes, fluorinated waxes, fluorinated gums, fatty acid esters chosen from ethyl and isopropyl palmitates, 2-ethylhexyl palmitate, 2-octyldecyl palmitate, butyl, cetyl or 2-octyldodecyl myristates, hexyl stearate, butyl stearate, dioctyl maleate, hexyl laurate, 2-hexyldecyl laurate and isononyl isononanate, insoluble silicones, amide compounds comprising at least one fatty chain chosen from those of the following formula (1) : 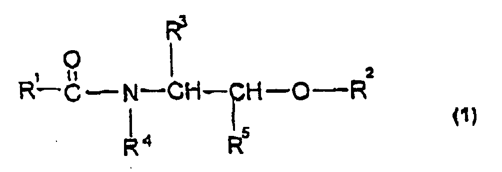
in which:
- R¹ denotes either a saturated or unsaturated, linear or branched, C₉-C₃₀ hydrocarbon radical, it being possible for this radical to be substituted with one or more hydroxyl groups optionally esterified with a saturated or unsaturated C₁₆-C₃₀ fatty acid; or a radical R"-(NR-CO)ₙ-R' in which n is equal to 0 or 1, R denotes hydrogen or hydroxyethyl, R' and R" are hydrocarbon radicals in which the sum of the carbon atoms is between 9 and 30, R' being a divalent radical,
- R² denotes a hydrogen atom or a (glycosyl)ₙ, (galactosyl)ₘ or sulphogalactosyl radical in which n is an integer varying from 1 to 4 and m is an integer varying from 1 to 8;
- R³ denotes a hydrogen atom or a saturated or unsaturated C₁₆-C₂₇ hydrocarbon radical, it being possible for this radical to be substituted with one or more C₁-C₁₄ alkyl radicals; R³ may also denote a C₁₅-C₂₆ α-hydroxyalkyl radical, the hydroxyl group being optionally esterified with a C₁₆-C₃₀ α-hydroxy acid;
- R⁴ denotes a hydrogen atom, a saturated or unsaturated C₁₆-C₂₇ hydrocarbon radical or a radical -CH₂-CHOH-CH₂-O-R⁶ in which R⁶ denotes a C₁₀-C₂₆ hydrocarbon radical;
- R⁵ denotes a hydrogen atom or a mono- or polyhydroxylated C₁-C₄ hydrocarbon radical;
it being possible for the said agents to be present in the form of mixtures;
**(c)** at least one oxidation dye,

2. Composition according to Claim 1, **characterized in that** the laccase(s) are chosen from laccases of plant origin, animal origin, fungal origin, bacterial origin or are obtained by biotechnology.

3. Composition according to either of Claims 1 to 2, where the laccases are chosen from those produced by plants performing chlorophyll synthesis.

4. Composition according to Claim 3, where the laccases are chosen from those extracted from Anacardiaceae or Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Indian pipe), Aesculus sp., Acer pseudoplatanus, Prunus persica, Pistacia palaestina.

5. Composition according to Claim 2, where the laccases are chosen from those derived from Pyricularia orizae, Polyporus versicolor, Rhizoctonia praticola, Rhus vernicifera, Scytalidium, Polyporus pinsitus, Myceliophtora thermophila, Rhizoctonia solani, Tramates versicolor, Fomes fomentarius, Chaetomium thermophile, Neurospora crassa, Coriolus versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitius squalens and variants thereof.

6. Composition according to any one of Claims 1 to 5, **characterized in that** the laccase(s) are provided in quantities ranging from 0.5 to 2000 lacu, or from 1000 to 4×10⁷ u units, or from 20 to 2×10⁶ lacu units, per 100 g of composition.

7. Composition according to Claim 1, according to which the conditioning agents are provided in liquid, semisolid or solid form and preferably in the form of an oil, wax or gum.

8. Composition according to Claim 1 or 7, according to which the conditioning agents are chosen from vegetable oils, natural waxes, insoluble silicones and the amide compounds of formula (1).

9. Composition according to Claim 1 or 7, according to which the poly-α-olefins are chosen from those:
- of the polybutene type, hydrogenated or otherwise, and preferably of the polyisobutene type, hydrogenated or otherwise;
- of the polydecene type, hydrogenated or otherwise.

10. Composition according to Claim 1 or 7, according to which the silicones are chosen from polydialkylsiloxanes, polyalkylarylsiloxanes, polydiaryldialkylsiloxanes which are optionally modified, preferably polydialkylsiloxanes or polyalkylarylsiloxanes, and still more preferably polydimethylsiloxanes which are optionally modified.

11. Composition according to Claim 10, where the silicones are chosen from polydialkylsiloxane gums, polyalkylarylsiloxane gums and are used alone or as mixtures in a solvent.

12. Composition according to any one of Claims 1, 7 or 8, **characterized in that** in formula (1) of the amide compounds, R¹ denotes a saturated or unsaturated alkyl derived from C₁₆-C₂₂ fatty acids; R² denotes a hydrogen atom; and R³ denotes a saturated linear C₁₅ radical.

13. Composition according to Claim 1 or 12, where the amide compounds are chosen from:
- N-linoleoyldihydrosphingosine,
- N-oleoyldihydrosphingosine,
- N-palmitoyldihydrosphingosine,
- N-stearoyldihydrosphingosine,
- N-behenoyldihydrosphingosine,
or the mixtures of these compounds.

14. Composition according to any one of Claims 1, 7 or 8, **characterized in that** in formula (1) of the amide compounds, R¹ denotes a saturated or unsaturated alkyl radical derived from fatty acids; R² denotes a galactosyl or sulphogalactosyl radical; and R³ denotes a group -CH=CH- (CH₂)₁₂-CH₃.

15. Composition according to any one of Claims 1 and 7 to 14, where the conditioning agents are present in proportions ranging from 0.001 to 10% by weight, preferably from 0.01 to 5% by weight, and still more particularly from 0.1 to 2% by weight relative to the total weight of the composition.

16. Composition according to Claim 1, **characterized in that** the oxidation dyes are oxidation bases chosen from ortho- or para-phenylenediamines, bisphenylalkylenediamines, ortho- or para-aminophenols, and heterocyclic bases, as well as the addition salts of these compounds with an acid.

17. Composition according to Claim 16, **characterized in that** the oxidation bases are present in concentrations ranging from 0.0005 to 12% by weight relative to the total weight of the composition.

18. Composition according to Claim 1, **characterized in that** the oxidation dyes are couplers chosen from meta-phenylenediamines, meta-aminophenols, meta-diphenols, heterocyclic couplers, and the addition salts of these compounds with an acid.

19. Composition according to Claim 18, **characterized in that** the couplers are present in concentrations ranging from 0.0001 to 10% by weight relative to the total weight of the composition.

20. Composition according to any one of Claims 1 and 16 to 19, **characterized in that** the addition salts with an acid of the dyes are chosen from hydrochlorides, hydrobromides, sulphates, tartrates, lactates and acetates.

21. Composition according to any one of the preceding claims, **characterized in that** it contains, in addition, direct dyes.

22. Composition according to any one of the preceding claims, **characterized in that** the medium appropriate for keratinous fibres (or carrier) consists of water or of a mixture of water and of at least one organic solvent.

23. Composition according to Claim 22, **characterized in that** the organic solvents may be present in proportions preferably ranging from 1 to 40% by weight relative to the total weight of the composition, and still more preferably ranging from 5 to 30% by weight.

24. Composition according to any one of the preceding claims, **characterized in that** the pH varies from 6 to 9 approximately.

25. Composition according to any one of the preceding claims, **characterized in that** it contains, in addition, at least one cosmetic adjuvant conventionally used in dyeing compositions, chosen from the group consisting of surfactants, polymers, thickening agents, antioxidants, enzymes different from laccases, penetrating agents, sequestering agents, perfumes, buffers, dispersing agents, film-forming agents, screening agents, vitamins, preservatives or opacifying agents.

26. Method of dyeing keratinous fibres, and in particular human keratinous fibres such as hair, **characterized in that** at least one ready-to-use dyeing composition as defined in any one of Claims 1 to 25 is applied to the said fibres for a sufficient time to develop the desired colour.

27. Method according to Claim 26, **characterized in that** it comprises a preliminary step consisting in storing in a separate form, on the one hand, a composition (A) comprising, in a medium appropriate for dyeing, at least one oxidation dye as defined in any one of Claims 1 and 16 to 20 and, on the other hand, a composition (B) containing, in a medium appropriate for keratinous fibres, at least one enzyme of the laccase type as defined in any one of Claims 1 to 6, and then in mixing them at the time of use before applying this mixture to the keratinous fibres; the composition (A) or the composition (B) containing the conditioning agent as defined in Claims 1 and 7 to 14.

28. Multicompartment device or dyeing "kit", **characterized in that** it comprises a first compartment containing the composition (A) as defined in Claim 27 and a second compartment containing the composition (B) as defined in Claim 27; the composition (A) or the composition (B) containing the conditioning agent as defined in Claims 1 and 7 to 14.

## Claims (Claims for the following Contracting State(s): BE, CH, CY, DE, ES, FR, GB, IT, NL)

1. Ready-to-use composition with a pH varying from 4 to 11 for the oxidation dyeing of keratinous fibres, in particular human keratinous fibres and more particularly human hair, comprising, in a carrier appropriate for dyeing keratinous fibres:
**(a)** at least one enzyme of the laccase type;
**(b)** at least one conditioning agent insoluble in aqueous media, chosen from the group consisting of:
poly-α-olefins, fluorinated oils, vegetable oils, natural waxes, fluorinated waxes, fluorinated gums, fatty acid esters chosen from ethyl and isopropyl palmitates, 2-ethylhexyl palmitate, 2-octyldecyl palmitate, butyl, cetyl or 2-octyldodecyl myristates, hexyl stearate, butyl stearate, dioctyl maleate, hexyl laurate, 2-hexyldecyl laurate and isononyl isononanate, insoluble silicones, amide compounds comprising at least one fatty chain chosen from those of the following formula (1): 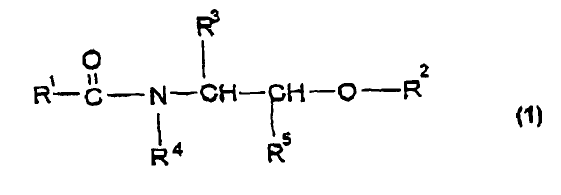
in which:
- R¹ denotes either a saturated or unsaturated, linear or branched, C₉-C₃₀ hydrocarbon radical, it being possible for this radical to be substituted with one or more hydroxyl groups optionally esterified with a saturated or unsaturated C₁₆-C₃₀ fatty acid; or a radical R"-(NR-CO)ₙ-R' in which n is equal to 0 or 1, R denotes hydrogen or hydroxyethyl, R' and R" are hydrocarbon radicals in which the sum of the carbon atoms is between 9 and 30, R' being a divalent radical,
- R² denotes a hydrogen atom or a (glycosyl)ₙ, (galactosyl)ₘ or sulphogalactosyl radical in which n is an integer varying from 1 to 4 and m is an integer varying from 1 to 8;
- R³ denotes a hydrogen atom or a saturated or unsaturated C₁₆-C₂₇ hydrocarbon radical, it being possible for this radical to be substituted with one or more C₁-C₁₄ alkyl radicals; R³ may also denote a C₁₅-C₂₆ α-hydroxyalkyl radical, the hydroxyl group being optionally esterified with a C₁₆-C₃₀ α-hydroxy acid;
- R⁴ denotes a hydrogen atom, a saturated or unsaturated C₁₆-C₂₇ hydrocarbon radical or a radical -CH₂-CHOH-CH₂-O-R⁶ in which R⁶ denotes a C₁₀-C₂₆ hydrocarbon radical;
- R⁵ denotes a hydrogen atom or a mono- or polyhydroxylated C₁-C₄ hydrocarbon radical;
it being possible for the said agents to be present in the form of mixtures;
**(c)** at least one oxidation dye;
excluding the following composition:
| | Quantity (% weight) |
|---|---|
| Toluene-2,5-diamine | 2.0 |
| Para-aminophenol | 1.0 |
| Meta-aminophenol | 0.03 |
| Laccase | 0.3 |
| Methylpolysiloxane (weight - average molecular weight: 100 000) | 4.5 |
| Methylpolysiloxane (30 cs) | 2.5 |
| N-cocoyl-L-glutamate triethanolamine | 0.5 |
| Yukaformer 301 | 0.9 |
| Ethanol | 5.0 |
| Pure water | qs 100 |
| Total | 100.0 |

2. Ready-to-use composition with a pH varying from 4 to 11 for the oxidation dyeing of keratinous fibres, in particular human keratinous fibres and more particularly human hair, comprising, in a carrier appropriate for dyeing keratinous fibres:
**(a)** at least one enzyme of the laccase type;
**(b)** at least one conditioning agent insoluble in aqueous media, chosen from the group consisting of:
poly-α-olefins; fluorinated oils; vegetable oils; natural waxes; fluorinated waxes; fluorinated gums; fatty acid esters chosen from ethyl and isopropyl palmitates, 2-ethylhexyl palmitate, 2-octyldecyl palmitate, butyl, cetyl or 2-octyldodecyl myristates, hexyl stearate, butyl stearate, dioctyl maleate, hexyl laurate, 2-hexyldecyl laurate and isononyl isononanate; insoluble silicones chosen from modified polydialkylsiloxanes, polyalkylarylsiloxanes, optionally modified polydiaryldialkylsiloxanes; amide compounds comprising at least one fatty chain chosen from those of the following formula (1): 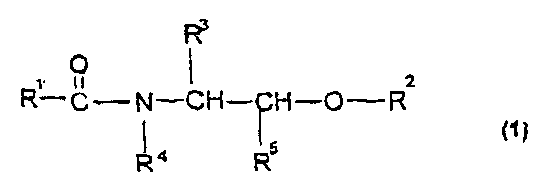
in which:
- R¹ denotes either a saturated or unsaturated, linear or branched, C₉-C₃₀ hydrocarbon radical, it being possible for this radical to be substituted with one or more hydroxyl groups optionally esterified with a saturated or unsaturated C₁₆-C₃₀ fatty acid; or a radical R"-(NR-CO)ₙ-R' in which n is equal to 0 or 1, R denotes hydrogen or hydroxyethyl, R' and R" are hydrocarbon radicals in which the sum of the carbon atoms is between 9 and 30, R' being a divalent radical,
- R² denotes a hydrogen atom or a (glycosyl)ₙ, (galactosyl)ₘ or sulphogalactosyl radical in which n is an integer varying from 1 to 4 and m is an integer varying from 1 to 8;
- R³ denotes a hydrogen atom or a saturated or unsaturated C₁₆-C₂₇ hydrocarbon radical, it being possible for this radical to be substituted with one or more C₁-C₁₄ alkyl radicals; R³ may also denote a C₁₅-C₂₆ α-hydroxyalkyl radical, the hydroxyl group being optionally esterified with a C₁₆-C₃₀ α-hydroxy acid;
- R⁴ denotes a hydrogen atom, a saturated or unsaturated C₁₆-C₂₇ hydrocarbon radical or a radical -CH₂-CHOH-CH₂-O-R⁶ in which R⁶ denotes a C₁₀-C₂₆ hydrocarbon radical;
- R⁵ denotes a hydrogen atom or a mono- or polyhydroxylated C₁-C₄ hydrocarbon radical;
it being possible for the said agents to be present in the form of mixtures;
**(c)** at least one oxidation dye.

3. Composition according to either of Claims 1 and 2, **characterized in that** the laccase(s) are chosen from laccases of plant origin, animal origin, fungal origin, bacterial origin or are obtained by biotechnology.

4. Composition according to any one of Claims 1 to 3, where the laccases are chosen from those produced by plants performing chlorophyll synthesis.

5. Composition according to Claim 4, where the laccases are chosen from those extracted from Anacardiaceae or Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Indian pipe), Aesculus sp., Acer pseudoplatanus, Prunus persica, Pistacia palaestina.

6. Composition according to Claim 3, where the laccases are chosen from those derived from Pyricularia orizae, Polyporus versicolor, Rhizoctonia praticola, Rhus vernicifera, Scytalidium, Polyporus pinsitus, Myceliophtora thermophila, Rhizoctonia solani, Tramates versicolor, Fomes fomentarius, Chaetomium thermophile, Neurospora crassa, Coriolus versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitius squalens and variants thereof.

7. Composition according to any one of Claims 1 to 6, **characterized in that** the laccase(s) are provided in quantities ranging from 0.5 to 2000 lacu, or from 1000 to 4×10⁷ u units or from 20 to 2×10⁶ lacu units, per 100 g of composition.

8. Composition according to Claim 1 or 2, according to which the conditioning agents are provided in liquid, semisolid or solid form and preferably in the form of an oil, wax or gum.

9. Composition according to Claims 1, 2 or 8, according to which the conditioning agents are chosen from vegetable oils, natural waxes, insoluble silicones and the amide compounds of formula (1).

10. Composition according to Claims 1, 2 or 8, according to which the poly-α-olefins are chosen from those:
- of the polybutene type, hydrogenated or otherwise, and preferably of the polyisobutene type, hydrogenated or otherwise;
- of the polydecene type, hydrogenated or otherwise.

11. Composition according to Claim 1 or 8, according to which the silicones are chosen from polydialkylsiloxanes, polyalkylarylsiloxanes, polydiaryldialkylsiloxanes which are optionally modified, preferably polydialkylsiloxanes or polyalkylarylsiloxanes, and still more preferably polydimethylsiloxanes which are optionally modified.

12. Composition according to Claim 11, where the silicones are chosen from polydialkylsiloxane gums, polyalkylarylsiloxane gums and are used alone or as mixtures in a solvent.

13. Composition according to Claim 2 or 8, according to which the silicones are chosen from modified polydialkylsiloxanes and optionally modified polyalkylarylsiloxanes.

14. Composition according to Claim 13, where the silicones are chosen from polyalkylarylsiloxane gums and are used alone or as mixtures in a solvent.

15. Composition according to any one of Claims 1, 2, 8 or 9, **characterized in that** in formula (1) of the amide compounds, R¹ denotes a saturated or unsaturated alkyl derived from C₁₆-C₂₂ fatty acids; R² denotes a hydrogen atom; and R³ denotes a saturated linear C₁₅ radical.

16. Composition according to Claim 1 or 15, where the amide compounds are chosen from:
- N-linoleoyldihydrosphingosine,
- N-oleoyldihydrosphingosine,
- N-palmitoyldihydrosphingosine,
- N-stearoyldihydrosphingosine,
- N-behenoyldihydrosphingosine,
or the mixtures of these compounds.

17. Composition according to any one of Claims 1, 2, 8 or 9, **characterized in that** in formula (1) of the amide compounds, R¹ denotes a saturated or unsaturated alkyl radical derived from fatty acids; R² denotes a galactosyl or sulphogalactosyl radical; and R³ denotes a group -CH=CH-(CH₂)₁₂-CH₃.

18. Composition according to any one of Claims 1, 2 and 8 to 17, where the conditioning agents are present in proportions ranging from 0.001 to 10% by weight, preferably from 0.01 to 5% by weight, and still more particularly from 0.1 to 2% by weight relative to the total weight of the composition.

19. Composition according to either of Claims 1 and 2, **characterized in that** the oxidation dyes are oxidation bases chosen from ortho- or para-phenylenediamines, bisphenylalkylenediamines, ortho- or para-aminophenols, and heterocyclic bases, as well as the addition salts of these compounds with an acid.

20. Composition according to Claim 19, **characterized in that** the oxidation bases are present in concentrations ranging from 0.0005 to 12% by weight relative to the total weight of the composition.

21. Composition according to either of Claims 1 and 2, **characterized in that** the oxidation dyes are couplers chosen from meta-phenylenediamines, meta-aminophenols, meta-diphenols, heterocyclic couplers, and the addition salts of these compounds with an acid.

22. Composition according to Claim 21, **characterized in that** the couplers are present in concentrations ranging from 0.0001 to 10% by weight relative to the total weight of the composition.

23. Composition according to any one of Claims 1 and 19 to 22, **characterized in that** the addition salts with an acid of the dyes are chosen from hydrochlorides, hydrobromides, sulphates, tartrates, lactates and acetates.

24. Composition according to any one of the preceding claims, **characterized in that** it contains, in addition, direct dyes.

25. Composition according to any one of the preceding claims, **characterized in that** the medium appropriate for keratinous fibres (or carrier) consists of water or of a mixture of water and of at least one organic solvent.

26. Composition according to Claim 25, **characterized in that** the organic solvents may be present in proportions preferably ranging from 1 to 40% by weight relative to the total weight of the composition, and still more preferably ranging from 5 to 30% by weight.

27. Composition according to any one of the preceding claims, **characterized in that** the pH varies from 6 to 9 approximately.

28. Composition according to any one of the preceding claims, **characterized in that** it contains, in addition, at least one cosmetic adjuvant conventionally used in dyeing compositions, chosen from the group consisting of surfactants, polymers, thickening agents, antioxidants, enzymes different from laccases, penetrating agents, sequestering agents, perfumes, buffers, dispersing agents, , film-forming agents, screening agents, vitamins, preservatives or opacifying agents.

29. Method of dyeing keratinous fibres, and in particular human keratinous fibres such as hair, **characterized in that** at least one ready-to-use dyeing composition as defined in any one of Claims 1 to 28 is applied to the said fibres for a sufficient time to develop the desired colour.

30. Method according to Claim 29, **characterized in that** it comprises a preliminary step consisting in storing in a separate form, on the one hand, a composition (A) comprising, in a medium appropriate for dyeing, at least one oxidation dye as defined in any one of Claims 1, 2 and 19 to 23 and, on the other hand, a composition (B) containing, in a medium appropriate for keratinous fibres, at least one enzyme of the laccase type as defined in any one of Claims 1 to 7, and then in mixing them at the time of use before applying this mixture to the keratinous fibres; the composition (A) or the composition (B) containing the conditioning agent as defined in any one of of Claims 1, 2 and 8 to 18.

31. Multicompartment device or dyeing "kit", **characterized in that** it comprises a first compartment containing the composition (A) as defined in Claim 30 and a second compartment containing the composition (B) as defined in Claim 30; the composition (A) or the composition (B) containing the conditioning agent as defined in Claims 1 and 8 to 18.

## Patentansprüche (Patentansprüche für folgende(n) Vertragsstaat(en): AT, DK, GR, IE, MC, PT, SE)

1. Gebrauchsfertige Zusammensetzung zum oxidativen Färben von Keratinfasern, insbesondere menschlichen Keratinfasern und besonders zum Färben menschlicher Haare mit einem pH-Wert von 4 bis 11, die in einem zum Färben von Keratinfasern geeigneten Träger enthält:
(a) mindestens ein Enzym vom Laccase-Typ;
(b) mindestens ein in wässrigen Medien unlösliches Konditioniermittel, das ausgewählt ist unter:
Poly-α-olefinen, fluorierten Ölen, pflanzlichen Ölen, natürlichen Wachsen, fluorierten Wachsen, fluorierten Gummis, Estern von Fettsäuren, die unter Ethylpalmitat, Isopropylpalmitat, 2-Ethylhexylpalmitat, 2-Octyldecylpalmitat, Butylmyristat, Cetylmyristat, 2-Octyldodecylmyristat, Hexylstearat, Butylstearat, Dioctylmalat, Hexyllaurat, 2-Hexyldecyllaurat und Isononylisononanoat ausgewählt sind, unlöslichen Siliconen, Amidverbindungen, die mindestens eine Fettkette aufweisen, die unter den Verbindungen der Formel (1) ausgewählt sind: 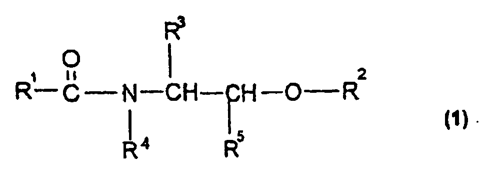
worin bedeuten:
- R¹ entweder eine geradkettige oder verzweigte, gesättigte oder ungesättigte C₉₋₃₀-Kohlenwasserstoffgruppe, wobei die Gruppe mit einer oder mehreren Hydroxygruppen substituiert sein kann, die gegebenenfalls mit einer gesättigten oder ungesättigten C₁₆₋₃₀-Fettsäure verestert sind; oder eine Gruppe R"-(NR-CO)ₙ-R', worin n 0 oder 1 bedeutet, R Wasserstoff oder Hydroxyethyl bedeutet und R' und R" Kohlenwasserstoffgruppen sind, wobei die Summe der Kohlenstoffatome der Gruppen R' und R" im Bereich von 9 bis 30 liegt und wobei R' eine zweiwertige Gruppe ist;
- R² Wasserstoff oder (Glycosyl)ₙ, (Galactosyl)ₘ oder Sulfogalactosyl, wobei n eine ganze Zahl im Bereich von 1 bis 4 und m eine ganze Zahl im Bereich von 1 bis 8 ist;
- R³ Wasserstoff oder eine gesättigte oder ungesättigte C₁₆₋₂₇₋Kohlenwasserstoffgruppe, wobei die Gruppe auch mit einer oder mehreren C₁₋₁₄-Alkylgruppen substituiert sein kann; R³ kann auch eine α-Hydroxyalkylgruppe mit 15 bis 26 Kohlenstoffatomen bedeuten, wobei die Hydroxygruppe gegebenenfalls mit einer α-Hydroxysäure mit 16 bis 30 Kohlenstoffatomen verestert sein kann;
- R⁴ Wasserstoff, eine gesättigte oder ungesättigte C₁₆₋₂₇₋Kohlenwasserstoffgruppe oder eine Gruppe -CH₂-CHOH-CH₂₋O-R₆, wobei R₆ eine C₁₀₋₂₆-Kohlenwasserstoffgruppe bedeutet;
- R⁵ Wasserstoff oder eine ein- oder mehrfach hydroxylierte C₁₋4-Kohlenwasserstoffgruppe;
wobei diese Stoffe auch im Gemisch enthalten sein können;
(c) mindestens einen Oxidationsfarbstoff.

2. Zusammensetzung nach Anspruch 1, **dadurch gekennzeichnet, dass** die Laccase(n) unter den Laccasen pflanzlicher Herkunft, den Laccasen tierischer Herkunft, den Laccasen, die von Pilzen gebildet werden, den Laccasen bakterieller Herkunft oder den biotechnologisch hergestellten Laccasen ausgewählt sind.

3. Zusammensetzung nach einem der Ansprüche 1 oder 2, wobei die Laccasen unter den Laccasen ausgewählt sind, die von Pflanzen gebildet werden, die Chlorophyll synthetisieren.

4. Zusammensetzung nach Anspruch 3, wobei die Laccasen unter den Laccasen ausgewählt sind, die aus Anacardiaceae oder Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Fichtenspargel), Aesculus sp., Acer pseudoplatanus, Prunus persica oder Pistacia palaestina extrahiert wurden.

5. Zusammensetzung nach Anspruch 2, wobei die Laccasen unter den Laccasen ausgewählt sind, die von Pyricularia oryzae, Polyporus versicolor, Rhizoctonia praticola, Rhus vernicifera, Scytalidium, Polyporus pinsitus, Myceliophtora thermophila, Rhizoctonia solani, Tramates versicolor, Fomes fomentarius, Chaetomium thermophile, Neurospora crassa, Coriolus versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitus squalens und deren Varianten stammen.

6. Zusammensetzung nach einem der Ansprüche 1 bis 5, **dadurch gekennzeichnet, dass** die Laccase(n) in einem Mengenanteil im Bereich von 0,5 bis 2000 lacu oder 1000 bis 4.10⁷ Einheiten u oder 20 bis 2.10⁶ Einheiten ulac pro 100 g Zusammensetzung vorliegen.

7. Zusammensetzung nach Anspruch 1, wobei die Konditioniermittel in flüssiger, halbfester oder fester Form vorliegen, insbesondere als Öl, Wachs oder Gummi.

8. Zusammensetzung nach den Ansprüchen 1 oder 7, wobei die Konditioniermittel unter den pflanzlichen Ölen, natürlichen Wachsen, unlöslichen Siliconen und Amiden der Formel (1) ausgewählt sind.

9. Zusammensetzung nach den Ansprüchen 1 oder 7, wobei die Poly-α-olefine unter den Verbindungen
- vom hydrierten oder nicht hydrierten Polybutentyp, vorzugsweise vom hydrierten oder nicht hydrierten Polyisobutentyp;
- vom hydrierten oder nicht hydrierten Polydecentyp ausgewählt sind.

10. Zusammensetzung nach den Ansprüchen 1 oder 7, wobei die Silicone unter den Polydialkylsiloxanen, Polyalkylarylsiloxanen, Polydiaryldialkylsiloxanen, die gegebenenfalls modifiziert sind, vorzugsweise Polydialkylsiloxanen oder Polyalkylarylsiloxanen und noch bevorzugter den gegebenenfalls modifizierten Polydimethylsiloxanen ausgewählt sind.

11. Zusammensetzung nach Anspruch 10, wobei die Silicone unter den Polydialkylsiloxangummis und Polyalkylarylsiloxangummis ausgewählt und alleine oder im Gemisch mit einem Lösungsmittel verwendet werden.

12. Zusammensetzung nach einem der Ansprüche 1, 7 oder 8, **dadurch gekennzeichnet, dass** bei den Amidverbindungen der Formel (1) R¹ eine gesättigte oder ungesättigte, von C₁₆₋₂₂-Fettsäuren abgeleitete Alkylgruppe, R² Wasserstoff und R³ eine gesättigte, geradkettige C₁₅-Gruppe bedeutet.

13. Zusammensetzung nach Anspruch 1 oder 12, wobei die Amide ausgewählt sind unter:
- N-Linoleoyldihydrosphingosin,
- N-Oleoyldihydrosphingosin,
- N-Palmitoyldihydrosphingosin,
- N-Stearoyldihydrosphingosin,
- N-Behenoyldihydrosphingosin, oder
- den Gemischen dieser Verbindungen.

14. Zusammensetzung nach einem der Ansprüche 1, 7 oder 8, **dadurch gekennzeichnet, dass** bei den Amidverbindungen der Formel (1) R¹ eine gesättigte oder ungesättigte, von Fettsäuren abgeleitete Alkylgruppe, R² eine Galactosyl- oder Sulfogalactosylgruppe und R³ die Gruppe -CH=CH-(CH₂)₁₂-CH₃ bedeutet.

15. Zusammensetzung nach einem der Ansprüche 1 und 7 bis 14, wobei die Konditioniermittel in Mengenanteilen von 0,001 bis 10 Gew.-%, vorzugsweise 0,01 bis 5 Gew.-% und insbesondere 0,1 bis 2 Gew.-%, bezogen auf das Gesamtgewicht der Zusammensetzung, enthalten sind.

16. Zusammensetzung nach Anspruch 1, **dadurch gekennzeichnet, dass** die Oxidationsfarbstoffe Oxidationsbasen sind, die unter den o- oder p-Phenylendiaminen, Bisphenylalkylendiaminen, o- oder p-Aminophenolen und den heterocyclischen Basen sowie den Additionsverbindungen dieser Verbindungen mit einer Säure ausgewählt sind.

17. Zusammensetzung nach Anspruch 16, **dadurch gekennzeichnet, dass** die Oxidationsbasen in Konzentrationen von 0,0005 bis 12 Gew.-%, bezogen auf das Gesamtgewicht der Zusammensetzung, vorliegen.

18. Zusammensetzung nach Anspruch 1, **dadurch gekennzeichnet, dass** die Oxidationsfarbstoffe Kuppler sind, die unter den m-Phenylendiaminen, m-Aminophenolen, m-Dihydroxybenzolen und heterocyclischen Kupplern und deren Additionssalzen mit einer Säure ausgewählt sind.

19. Zusammensetzung nach Anspruch 18, **dadurch gekennzeichnet, dass** die Kuppler in Konzentrationen von 0,0001 bis 10 Gew.-%, bezogen auf das Gesamtgewicht der Zusammensetzung, vorliegen.

20. Zusammensetzung nach einem der Ansprüche 1 und 16 bis 19, **dadurch gekennzeichnet, dass** die Additionssalze der Oxidationsfarbstoffe mit einer Säure unter den Hydrochloriden, Hydrobromiden, Sulfaten, Tartraten, Lactaten und Acetaten ausgewählt sind.

21. Zusammensetzung nach einem der vorhergehenden Ansprüche, **dadurch gekennzeichnet, dass** sie ferner Direktfarbstoffe enthält.

22. Zusammensetzung nach einem der vorhergehenden Ansprüche, **dadurch gekennzeichnet, dass** das für Keratinfasern geeignete Medium (oder der Träger) aus Wasser oder einem Gemisch von Wasser und mindestens einem organischen Lösungsmittel besteht.

23. Zusammensetzung nach Anspruch 22, **dadurch gekennzeichnet, dass** die organischen Lösungsmittel vorzugsweise in Mengenanteilen von etwa 1 bis 40 Gew.-%, bezogen auf das Gesamtgewicht der Zusammensetzung, und noch bevorzugter 5 bis 30 Gew.-% vorliegen können.

24. Zusammensetzung nach einem der vorhergehenden Ansprüche, **dadurch gekennzeichnet, dass** der pH-Wert im Bereich von 6 bis 9 liegt.

25. Zusammensetzung nach einem der vorhergehenden Ansprüche, **dadurch gekennzeichnet, dass** sie ferner mindestens einen herkömmlich in Zusammensetzungen zum Färben der Haare verwendeten kosmetischen Zusatzstoff enthält, der unter den grenzflächenaktiven Stoffen, Polymeren, Verdickungsmitteln, Antioxidantien, Enzymen, die von Laccasen verschieden sind, Penetrationsmitteln, Maskierungsmitteln, Parfums, Puffern, Dispergiermitteln, Filmbildnern, Filtern, Vitaminen, Konservierungsmitteln und Trübungsmitteln ausgewählt ist.

26. Verfahren zum Färben von Keratinfasern und insbesondere menschlichen Keratinfasern, wie Haaren, **dadurch gekennzeichnet, dass** auf die Fasern während einer Zeitspanne, die zur Entwicklung der gewünschten Färbung ausreichend ist, mindestens eine gebrauchsfertige Farbmittelzusammensetzung nach einem der Ansprüche 1 bis 25 aufgebracht wird.

27. Verfahren nach Anspruch 26, **dadurch gekennzeichnet, dass** es einen vorbereitenden Schritt umfasst, der darin besteht, einerseits eine Zusammensetzung (A), die in einem zum Färben geeigneten Medium mindestens einen Oxidationsfarbstoff nach einem der Ansprüche 1 und 16 bis 20 enthält, und andererseits eine Zusammensetzung (B) getrennt voneinander aufzubewahren, die in einem für Keratinfasern geeigneten Medium mindestens ein Enzym vom Laccase-Typ nach einem der Ansprüche 1 bis 6 enthält, und diese bei der Anwendung zu vermischen, bevor das Gemisch auf die Keratinfasern aufgebracht wird, wobei die Zusammensetzung (A) oder die Zusammensetzung (B) das Konditioniermittel nach einem der Ansprüche 1 und 7 bis 14 enthält.

28. Vorrichtung mit mehreren Abteilungen oder "Kit" zum Färben, **dadurch gekennzeichnet, dass** sie eine erste Abteilung mit der in Anspruch 27 definierten Zusammensetzung (A) und eine zweite Abteilung mit der in Anspruch 27 definierten Zusammensetzung enthält, wobei die Zusammensetzung (A) oder die Zusammensetzung (B) das Konditioniermittel nach einem der Ansprüche 1 und 7 bis 14 enthält.

## Patentansprüche (Patentansprüche für folgende(n) Vertragsstaat(en): BE, CH, LI, DE, ES, FR, GB, IT, NL)

1. Gebrauchsfertige Zusammensetzung zum oxidativen Färben von Keratinfasern, insbesondere menschlichen Keratinfasern und besonders zum Färben menschlicher Haare mit einem pH-Wert von 4 bis 11, die in einem zum Färben von Keratinfasern geeigneten Träger enthält:
(a) mindestens ein Enzym vom Laccase-Typ;
(b) mindestens ein in wässrigen Medien unlösliches Konditioniermittel, das ausgewählt ist unter:
Poly-α-olefinen, fluorierten Ölen, pflanzlichen Ölen, natürlichen Wachsen, fluorierten Wachsen, fluorierten Gummis, Estern von Fettsäuren, die unter Ethylpalmitat, Isopropylpalmitat, 2-Ethylhexylpalmitat, 2-Octyldecylpalmitat, Butylmyristat, Cetylmyristat, 2-Octyldodecylmyristat, Hexylstearat, Butylstearat, Dioctylmalat, Hexyllaurat, 2-Hexyldecyllaurat und Isononylisononanoat ausgewählt sind, unlöslichen Siliconen, Amidverbindungen, die mindestens eine Fettkette aufweisen, die unter den Verbindungen der Formel (1) ausgewählt sind: 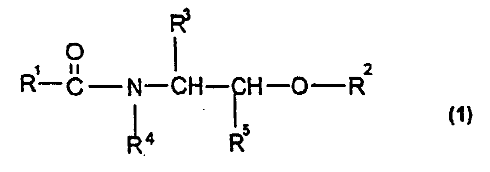
worin bedeuten:
- R¹ entweder eine geradkettige oder verzweigte, gesättigte oder ungesättigte C₉₋₃₀-Kohlenwasserstoffgruppe, wobei die Gruppe mit einer oder mehreren Hydroxygruppen substituiert sein kann, die gegebenenfalls mit einer gesättigten oder ungesättigten C₁₆₋₃₀-Fettsäure verestert sind; oder eine Gruppe R"-(NR-CO)ₙ-R', worin n 0 oder 1 bedeutet, R Wasserstoff oder Hydroxyethyl bedeutet und R' und R" Kohlenwasserstoffgruppen sind, wobei die Summe der Kohlenstoffatome der Gruppen R' und R" im Bereich von 9 bis 30 liegt und wobei R' eine zweiwertige Gruppe ist;
- R² Wasserstoff oder (Glycosyl)ₙ, (Galactosyl)ₘ oder Sulfogalactosyl, wobei n eine ganze Zahl im Bereich von 1 bis 4 und m eine ganze Zahl im Bereich von 1 bis 8 ist;
- R³ Wasserstoff oder eine gesättigte oder ungesättigte C₁₆₋₂₇₋Kohlenwasserstoffgruppe, wobei die Gruppe auch mit einer oder mehreren C₁₋₁₄-Alkylgruppen substituiert sein kann; R³ kann auch eine α-Hydroxyalkylgruppe mit 15 bis 26 Kohlenstoffatomen bedeuten, wobei die Hydroxygruppe gegebenenfalls mit einer α-Hydroxysäure mit 16 bis 30 Kohlenstoffatomen verestert sein kann;
- R⁴ Wasserstoff, eine gesättigte oder ungesättigte C₁₆₋₂₇-Kohlenwasserstoffgruppe oder eine Gruppe -CH₂-CHOH-CH₂-O-R₆, wobei R₆ eine C₁₀₋₂₆-Kohlenwasserstoffgruppe bedeutet;
- R⁵ Wasserstoff oder eine ein- oder mehrfach hydroxylierte C₁₋₄-Kohlenwasserstoffgruppe;
wobei diese Stoffe auch im Gemisch enthalten sein können;
(c) mindestens einen Oxidationsfarbstoff;
wobei die folgende Zusammensetzung ausgenommen ist:
| | Menge (Gew.-%) |
|---|---|
| Toluol-2,5-diamin | 2,0 |
| *p*-Aminophenol | 1,0 |
| *m*-Aminophenol | 0,03 |
| Laccase | 0,3 |
| Methylpolysiloxan (mittleres Molekulargewicht: 100.000) | 4,5 |
| Methylpolysiloxan (30 cS) | 2,5 |
| Triethanolamin-N-cocoyl-L-glutamat | 0,5 |
| Yukaformer 301 | 0,9 |
| Ethanol | 5,0 |
| reines Wasser | ad 100 |
| Insgesamt | 100,0 |

2. Gebrauchsfertige Zusammensetzung zum oxidativen Färben von Keratinfasern, insbesondere menschlichen Keratinfasern und besonders zum Färben menschlicher Haare mit einem pH-Wert von 4 bis 11, die in einem zum Färben von Keratinfasern geeigneten Träger enthält:
(a) mindestens ein Enzym vom Laccase-Typ;
(b) mindestens ein in wässrigen Medien unlösliches Konditioniermittel, das ausgewählt ist unter:
Poly-α-olefinen, fluorierten Ölen, pflanzlichen Ölen, natürlichen Wachsen, fluorierten Wachsen, fluorierten Gummis, Estern von Fettsäuren, die unter Ethylpalmitat, Isopropylpalmitat, 2-Ethylhexylpalmitat, 2-Octyldecylpalmitat, Butylmyristat, Cetylmyristat, 2-Octyldodecylmyristat, Hexylstearat, Butylstearat, Dioctylmalat, Hexyllaurat, 2-Hexyldecyllaurat und Isononylisononanoat ausgewählt sind, unlöslichen Siliconen, die unter den modifizierten Polydialkylsiloxanen, Polyalkylarylsiloxanen, Polydiaryldialkylsiloxanen, die gegebenenfalls modifiziert sind, ausgewählt sind; Amidverbindungen, die mindestens eine Fettkette aufweisen, die unter den Verbindungen der Formel (1) ausgewählt sind: 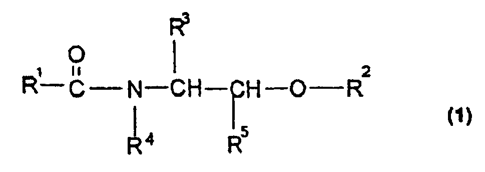
worin bedeuten:
- R¹ entweder eine geradkettige oder verzweigte, gesättigte oder ungesättigte C₉₋₃₀-Kohlenwasserstoffgruppe, wobei die Gruppe mit einer oder mehreren Hydroxygruppen substituiert sein kann, die gegebenenfalls mit einer gesättigten oder ungesättigten C₁₆₋₃₀-Fettsäure verestert sind; oder eine Gruppe R"-(NR-CO)ₙ-R', worin n 0 oder 1 bedeutet, R Wasserstoff oder Hydroxyethyl bedeutet und R' und R" Kohlenwasserstoffgruppen sind, wobei die Summe der Kohlenstoffatome der Gruppen R' und R" im Bereich von 9 bis 30 liegt und wobei R' eine zweiwertige Gruppe ist;
- R² Wasserstoff oder (Glycosyl)ₙ, (Galactosyl)ₘ oder Sulfogalactosyl, wobei n eine ganze Zahl im Bereich von 1 bis 4 und m eine ganze Zahl im Bereich von 1 bis 8 ist;
- R³ Wasserstoff oder eine gesättigte oder ungesättigte C₁₆₋₂₇₋Kohlenwasserstoffgruppe, wobei die Gruppe auch mit einer oder mehreren C₁₋₁₄-Alkylgruppen substituiert sein kann; R³ kann auch eine α-Hydroxyalkylgruppe mit 15 bis 26 Kohlenstoffatomen bedeuten, wobei die Hydroxygruppe gegebenenfalls mit einer α-Hydroxysäure mit 16 bis 30 Kohlenstoffatomen verestert sein kann;
- R⁴ Wasserstoff, eine gesättigte oder ungesättigte C₁₆₋₂₇₋Kohlenwasserstoffgruppe oder eine Gruppe -CH₂-CHOH-CH₂₋O-R₆, wobei R₆ eine C₁₀₋₂₆-Kohlenwasserstoffgruppe bedeutet;
- R⁵ Wasserstoff oder eine ein- oder mehrfach hydroxylierte C₁₋₄-Kohlenwasserstoffgruppe;
wobei diese Stoffe auch im Gemisch enthalten sein können;
(c) mindestens einen Oxidationsfarbstoff;

3. Zusammensetzung nach Anspruch 1 bis 2, **dadurch gekennzeichnet, dass** die Laccase(n) unter den Laccasen pflanzlicher Herkunft, den Laccasen tierischer Herkunft, den Laccasen, die von Pilzen gebildet werden, den Laccasen bakterieller Herkunft oder den biotechnologisch hergestellten Laccasen ausgewählt sind.

4. Zusammensetzung nach einem der Ansprüche 1 bis 3, wobei die Laccasen unter den Laccasen ausgewählt sind, die von Pflanzen gebildet werden, die Chlorophyll synthetisieren.

5. Zusammensetzung nach Anspruch 4, wobei die Laccasen unter den Laccasen ausgewählt sind, die aus Anacardiaceae oder Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Fichtenspargel), Aesculus sp., Acer pseudoplatanus, Prunus persica oder Pistacia palaestina extrahiert wurden.

6. Zusammensetzung nach Anspruch 3, wobei die Laccasen unter den Laccasen ausgewählt sind, die von Pyricularia oryzae, Polyporus versicolor, Rhizoctonia praticola, Rhus vernicifera, Scytalidium, Polyporus pinsitus, Myceliophtora thermophila, Rhizoctonia solani, Tramates versicolor, Fomes fomentarius, Chaetomium thermophile, Neurospora crassa, Coriolus versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitus squalens und deren Varianten stammen.

7. Zusammensetzung nach einem der Ansprüche 1 bis 6, **dadurch gekennzeichnet, dass** die Laccase(n) in einem Mengenanteil im Bereich von 0,5 bis 2000 lacu oder 1000 bis 4.10⁷ Einheiten u oder 20 bis 2.10⁶ Einheiten ulac pro 100 g Zusammensetzung vorliegen.

8. Zusammensetzung nach Anspruch 1 oder 2, wobei die Konditioniermittel in flüssiger, halbfester oder fester Form vorliegen, insbesondere als Öl, Wachs oder Gummi.

9. Zusammensetzung nach den Ansprüchen 1, 2 oder 8, wobei die Konditioniermittel unter den pflanzlichen Ölen, natürlichen Wachsen, unlöslichen Siliconen und Amiden der Formel (1) ausgewählt sind.

10. Zusammensetzung nach den Ansprüchen 1, 2 oder 8, wobei die Poly-α-olefine unter den Verbindungen
- vom hydrierten oder nicht hydrierten Polybutentyp, vorzugsweise vom hydrierten oder nicht hydrierten Polyisobutentyp;
- vom hydrierten oder nicht hydrierten Polydecentyp ausgewählt sind.

11. Zusammensetzung nach den Ansprüchen 1 oder 8, wobei die Silicone unter den Polydialkylsiloxanen, Polyalkylarylsiloxanen, Polydiaryldialkylsiloxanen, die gegebenenfalls modifiziert sind, vorzugsweise Polydialkylsiloxanen oder Polyalkylarylsiloxanen und noch bevorzugter den gegebenenfalls modifizierten Polydimethylsiloxanen ausgewählt sind.

12. Zusammensetzung nach Anspruch 11, wobei die Silicone unter den Polydialkylsiloxangummis und Polyalkylarylsiloxangummis ausgewählt und alleine oder im Gemisch mit einem Lösungsmittel verwendet werden.

13. Zusammensetzung nach den Ansprüchen 2 oder 8, wobei die Silicone unter den modifizierten Polydialkylsiloxanen und den Polyalkylarylsiloxanen, die gegebenenfalls modifiziert sind, ausgewählt sind.

14. Zusammensetzung nach Anspruch 13, wobei die Silicone unter den Polyalkylarylsiloxangummis ausgewählt sind und alleine oder im Gemisch mit einem Lösungsmittel verwendet werden.

15. Zusammensetzung nach einem der Ansprüche 1, 2, 8 oder 9, **dadurch gekennzeichnet, dass** bei den Amidverbindungen der Formel (1) R¹ eine gesättigte oder ungesättigte, von C₁₆₋₂₂₋Fettsäuren abgeleitete Alkylgruppe, R² Wasserstoff und R³ eine gesättigte, geradkettige C₁₅-Gruppe bedeutet.

16. Zusammensetzung nach Anspruch 1 oder 15, wobei die Amide ausgewählt sind unter:
- N-Linoleoyldihydrosphingosin,
- N-Oleoyldihydrosphingosin,
- N-Palmitoyldihydrosphingosin,
- N-Stearoyldihydrosphingosin,
- N-Behenoyldihydrosphingosin, oder
- den Gemischen dieser Verbindungen.

17. Zusammensetzung nach einem der Ansprüche 1, 2, 8 oder 9, **dadurch gekennzeichnet, dass** bei den Amidverbindungen der Formel (1) R¹ eine gesättigte oder ungesättigte, von Fettsäuren abgeleitete Alkylgruppe, R² eine Galactosyl- oder Sulfogalactosylgruppe und R³ die Gruppe -CH=CH-(CH₂)₁₂-CH₃ bedeutet.

18. Zusammensetzung nach einem der Ansprüche 1, 2 und 8 bis 17,
wobei die Konditioniermittel in Mengenanteilen von 0,001 bis 10 Gew.-%, vorzugsweise 0,01 bis 5 Gew.-% und insbesondere 0,1 bis 2 Gew.-%, bezogen auf das Gesamtgewicht der Zusammensetzung, enthalten sind.

19. Zusammensetzung nach einem der Ansprüche 1 oder 2, **dadurch gekennzeichnet, dass** die Oxidationsfarbstoffe Oxidationsbasen sind, die unter den o- oder p-Phenylendiaminen, Bisphenylalkylendiaminen, o- oder p-Aminophenolen und den heterocyclischen Basen sowie den Additionsverbindungen dieser Verbindungen mit einer Säure ausgewählt sind.

20. Zusammensetzung nach Anspruch 19, **dadurch gekennzeichnet, dass** die Oxidationsbasen in Konzentrationen von 0,0005 bis 12 Gew.-%, bezogen auf das Gesamtgewicht der Zusammensetzung, vorliegen.

21. Zusammensetzung nach Anspruch 1 oder 2, **dadurch gekennzeichnet, dass** die Oxidationsfarbstoffe Kuppler sind, die unter den m-Phenylendiaminen, m-Aminophenolen, m-Dihydroxybenzolen und heterocyclischen Kupplern und deren Additionssalzen mit einer Säure ausgewählt sind.

22. Zusammensetzung nach Anspruch 21, **dadurch gekennzeichnet, dass** die Kuppler in Konzentrationen von 0,0001 bis 10 Gew.-%, bezogen auf das Gesamtgewicht der Zusammensetzung, vorliegen.

23. Zusammensetzung nach einem der Ansprüche 1 und 19 bis 22, **dadurch gekennzeichnet, dass** die Additionssalze der Oxidationsfarbstoffe mit einer Säure unter den Hydrochloriden, Hydrobromiden, Sulfaten, Tartraten, Lactaten und Acetaten ausgewählt sind.

24. Zusammensetzung nach einem der vorhergehenden Ansprüche, **dadurch gekennzeichnet, dass** sie ferner Direktfarbstoffe enthält.

25. Zusammensetzung nach einem der vorhergehenden Ansprüche, **dadurch gekennzeichnet, dass** das für Keratinfasern geeignete Medium (oder der Träger) aus Wasser oder einem Gemisch von Wasser und mindestens einem organischen Lösungsmittel besteht.

26. Zusammensetzung nach Anspruch 25, **dadurch gekennzeichnet, dass** die organischen Lösungsmittel vorzugsweise in Mengenanteilen von etwa 1 bis 40 Gew.-%, bezogen auf das Gesamtgewicht der Zusammensetzung, und noch bevorzugter 5 bis 30 Gew.-% vorliegen können.

27. Zusammensetzung nach einem der vorhergehenden Ansprüche, **dadurch gekennzeichnet, dass** der pH-Wert im Bereich von 6 bis 9 liegt.

28. Zusammensetzung nach einem der vorhergehenden Ansprüche, **dadurch gekennzeichnet, dass** sie ferner mindestens einen herkömmlich in Zusammensetzungen zum Färben der Haare verwendeten kosmetischen Zusatzstoff enthält, der unter den grenzflächenaktiven Stoffen, Polymeren, Verdickungsmitteln, Antioxidantien, Enzymen, die von Laccasen verschieden sind, Penetrationsmitteln, Maskierungsmitteln, Parfums, Puffern, Dispergiermitteln, Filmbildnern, Filtern, Vitaminen, Konservierungsmitteln und Trübungsmitteln ausgewählt ist.

29. Verfahren zum Färben von Keratinfasern und insbesondere menschlichen Keratinfasern, wie Haaren, **dadurch gekennzeichnet, dass** auf die Fasern während einer Zeitspanne, die zur Entwicklung der gewünschten Färbung ausreichend ist, mindestens eine gebrauchsfertige Farbmittelzusammensetzung nach einem der Ansprüche 1 bis 28 aufgebracht wird.

30. Verfahren nach Anspruch 29, **dadurch gekennzeichnet, dass** es einen vorbereitenden Schritt umfasst, der darin besteht, einerseits eine Zusammensetzung (A), die in einem zum Färben geeigneten Medium mindestens einen Oxidationsfarbstoff nach einem der Ansprüche 2 und 19 bis 23 enthält, und andererseits eine Zusammensetzung (B) getrennt voneinander aufzubewahren, die in einem für Keratinfasern geeigneten Medium mindestens ein Enzym vom Laccase-Typ nach einem der Ansprüche 1 bis 7 enthält, und diese bei der Anwendung zu vermischen, bevor das Gemisch auf die Keratinfasern aufgebracht wird, wobei die Zusammensetzung (A) oder die Zusammensetzung (B) das Konditioniermittel nach einem der Ansprüche 1, 2 und 8 bis 18 enthält.

31. Vorrichtung mit mehreren Abteilungen oder "Kit" zum Färben, **dadurch gekennzeichnet, dass** sie eine erste Abteilung mit der in Anspruch 30 definierten Zusammensetzung (A) und eine zweite Abteilung mit der in Anspruch 30 definierten Zusammensetzung enthält, wobei die Zusammensetzung (A) oder die Zusammensetzung (B) das Konditioniermittel nach einem der Ansprüche 1 und 8 bis 18 enthält.
